(19) Europäisches Patentamt
European Patent Office
Office européen des brevets

(11) **EP 2 664 280 A2**

(12) **EUROPEAN PATENT APPLICATION**

(43) Date of publication:
**20.11.2013 Bulletin 2013/47**

(51) Int Cl.:
*A61B 6/00* (2006.01)　　*H05G 1/60* (2006.01)
*G01T 7/00* (2006.01)　　*G01T 1/36* (2006.01)
*G01V 5/00* (2006.01)　　*H01L 27/146* (2006.01)

(21) Application number: **13167673.6**

(22) Date of filing: **14.05.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.05.2012　US 201261646488 P
31.12.2012　KR 20120158483**

(71) Applicant: **Samsung Electronics Co., Ltd
Gyeonggi-do 443-742 (KR)**

(72) Inventors:
• **Kang, Dong Goo**
  **Gyeonggi-do (KR)**
• **Kang, Sung Hoon**
  **Gyeonggi-do (KR)**
• **Sung, Young Hun**
  **Gyeonggi-do (KR)**

(74) Representative: **Grünecker, Kinkeldey,
Stockmair & Schwanhäusser
Leopoldstrasse 4
80802 München (DE)**

(54) **X-ray imaging apparatus and control method therefor**

(57)　　Disclosed herein are an X-ray imaging apparatus for forming an X-ray image having reduced noise by correcting errors according to characteristics of each of a plurality of pixels and a control method therefor. The X-ray imaging apparatus includes an X-ray generator to generate X-rays and irradiate the generated X-rays, an X-ray detector to detect the irradiated X-rays and output X-ray data by counting the number of photons having an energy that is equal to or greater than threshold energy among photons contained in the detected X-rays, for each of a plurality of pixels; a function acquisition unit to acquire calibration functions for the respective pixels using X-ray data output for a plurality of predesigned phantoms, and an image correction unit to correct an X-ray image of an object on a per pixel basis using the acquired calibration functions.

FIG.7

**Description**

BACKGROUND

**1. Field**

Exemplary embodiments consistent with the present invention relate to an X-ray imaging apparatus for performing X-ray imaging by transmitting X-rays to an object and a control method therefor.

**2. Description of the Related Art**

X-ray imaging apparatuses are devices which irradiate an object with X-rays and acquire an image inside the object using X-rays transmitted through the object. A structure inside the object may be imaged by detecting the intensity of the X-rays transmitted through the object because transmittance of X-rays varies according to characteristics of materials constituting the object.

In particular, when an X-ray generator generates X-rays and irradiates an object with the generated X-rays, and an X-ray detector detects X-rays transmitted through the object and converts the detected X-rays into an electrical signal. Conversion into an electrical signal may be performed for each of a plurality of pixels and thus electrical signals corresponding to the respective pixels are combined, thereby acquiring a single X-ray image.

Conventionally, a method of reading out electrical signals after being accumulated for a certain period of time has been widely used. Recently, however, a photon counting detector (PCD) for sorting detected X-rays according to energies by counting photons with a certain energy level or higher has been developed.

The PCD may separate a particular material from an X-ray image and is advantageous in terms of less X-ray exposure and noise. However, the PCD is affected by characteristics of a light receiving element for each pixel or characteristics of a read-out circuit. Thus, even though X-rays with the same energy are irradiated to all the pixels, different counter values for each pixel may be output, which may cause generation of noise in an image.

SUMMARY

Therefore, it is an aspect of the exemplary embodiments to provide an X-ray imaging apparatus for forming an X-ray image with reduced noise by correcting errors according to characteristics of each pixel and a method for controlling the X-ray imaging apparatus.

Additional aspects of the invention will be set forth in part in the description which follows and, in part, will be obvious from the description, or may be learned by practice of the invention.

In accordance with one aspect of the exemplary embodiments, it is provided an X-ray imaging apparatus including an X-ray generator to generate X-rays and irradiate the generated X-rays, an X-ray detector to detect the X-rays and output X-ray data by counting the number of photons having an energy that is equal to or greater than threshold energy among photons contained in the detected X-rays, for each of a plurality of pixels, a function acquisition unit to determine calibration functions for the respective pixels using X-ray data for a plurality of predesigned phantoms, and an image correction unit to correct an X-ray image of an object on a per pixel basis using the acquired calibration functions. Thus errors on a per pixel basis may be corrected by applying the calibration functions for correction of each pixel.

The function acquisition unit may include a measurement value storage unit to store X-ray data output by the X-ray detector after the X-ray generator irradiates the phantoms with X-rays and the X-ray detector detects X-rays transmitted through the phantoms, and a calculator to perform calculation to acquire the calibration functions using the X-ray data stored in the measurement value storage unit.

Thus radiography may be performed on the designed phantoms under radiography conditions of an actual object to measure X-ray data. The radiography may be performed for sufficient X-ray exposure time to minimize impact of quantum noise.

The function acquisition unit may divide the phantoms into at least two phantom sets and acquire the calibration functions for each phantom set.

Thus, a phantom set may comprise different types of phantoms belonging to different sets of phantoms, the set representing different radiography conditions.

The image correction unit may produce at least two corrected X-ray images by applying the calibration functions acquired for each phantom set to the X-ray image of the object.

The image correction unit may produce a single corrected X-ray image by composing the at least two corrected X-ray images.

Thus the image correction unit may compose the corrected images, which are obtained using different calibration functions, to form a corrected final image.

In accordance with another aspect of the exemplary embodiments, it is provided a method for controlling an X-ray imaging apparatus the method including acquiring a calibration function for each of a plurality of pixels using X-ray data for a  plurality of predesigned phantoms, irradiating an object with X-rays and detecting X-rays transmitted through the object to acquire an X-ray image of the object, and correcting the X-ray image of the object on a per pixel basis using the acquired calibration function.
The advantages of the method are the same as discussed above with respect to the X-ray imaging apparatus.

In accordance with an aspect of the exemplary embodiments, there is provided an X-ray imaging apparatus including: an X-ray generator configured to generate X-rays and irradiate the generated X-rays toward an object; an X-ray detector configured to detect the irradiated X-rays and output X-ray data by counting a number of photons having an energy level that is equal to or greater than a threshold energy level, among photons contained in the detected X-rays, for each of a plurality of pixels of the X-ray detector; a function acquisition unit configured to determine calibration functions for the plurality of pixels using X-ray data obtained from a plurality of predesigned phantoms; and an image correction unit configured to correct an X-ray image of the object on a per pixel basis using the determined calibration functions.
The advantages of said apparatus are the same as discussed above with respect to the X-ray imaging apparatus.

In accordance with another aspect of the exemplary embodiments, there is provided a method of controlling an X-ray imaging apparatus, the method including: determining calibration functions for a plurality of pixels using X-ray data obtained from a plurality of predesigned phantoms; irradiating an object with X-rays and detecting X-rays transmitted through the object to acquire an X-ray image of the  object; and correcting the X-ray image of the object on a per pixel basis using the determined calibration functions.
The advantages of the method are the same as discussed above with respect to the X-ray imaging apparatus.

According to yet another aspect of the exemplary embodiments, there is provided a method of processing an X-ray image, the method including: calibrating an x-ray detector, the calibrating including calculating calibration functions for a plurality of image areas by correlating measured X-ray data obtained from a plurality of predetermined imaging phantoms to estimated x-ray data for the plurality of predetermined imaging phantoms; emitting X-rays toward an object and detecting X-rays transmitted through the object to capture an X-ray image of the object; and adjusting the X-ray image of the object in units of image areas based on the calculated calibration functions.
The advantages of the method are the same as discussed above with respect to the X-ray imaging apparatus.

BRIEF DESCRIPTION OF THE DRAWINGS

These and/or other aspects of the invention will become apparent and more readily appreciated from the following description of the exemplary embodiments, taken in conjunction with the accompanying drawings of which:

FIG. 1 is a general view of an X-ray imaging apparatus for mammography, according to an exemplary embodiment;

FIG. 2 is a control block diagram of the X-ray imaging apparatus according to an exemplary embodiment;

FIG. 3 is a schematic diagram illustrating a structure of an X-ray detector of the X-ray imaging apparatus according to an exemplary embodiment;

FIG. 4A is a schematic diagram illustrating a structure of a single pixel region of the X-ray detector illustrated in FIG. 3;

FIG. 4B is a schematic diagram illustrating a structure of a single pixel region in which detected X-rays are divided according to a plurality of energy bands;

FIG. 5A is a graph showing an energy spectrum of X-rays irradiated by an X-ray generator, according to an exemplary embodiment;

FIG. 5B is a graph showing ideal spectrum for a case in which the X-ray detector of FIG. 4A divides X-rays according to energy bands, according to an exemplary embodiment;

FIG. 6 is a graph showing a plot of a measured X-ray intensity normalized for each threshold energy and a theoretical plot thereof, according to an exemplary embodiment;

FIG. 7 is a control block diagram specifically illustrating a structure of a controller of the X-ray imaging apparatus according to an exemplary embodiment;

FIG. 8A is a schematic view of X-ray data output from each pixel of the X-ray detector of the X-ray imaging apparatus according to an exemplary embodiment;

FIG. 8B is a schematic view of data stored by a function acquisition unit of the X-ray imaging apparatus according to an exemplary embodiment;

FIG. 9 is a schematic view of a structure of data stored in a function storage unit, according to an exemplary embodiment;

FIG. 10 is a schematic view of a structure of data stored in a measurement value storage unit when calibration functions are acquired for each condition, according to an exemplary embodiment;

FIG. 11 is a schematic view of a structure of data stored in a function storage unit when calibration functions are acquired for each condition, according to an exemplary embodiment;

FIG. 12 is a control block diagram of an X-ray imaging apparatus according to an exemplary embodiment;

FIG. 13 is a schematic view illustrating a structure of data stored in a function storage unit of the X-ray imaging apparatus according to an exemplary embodiment illustrated in FIG. 12;

FIG. 14 is a sectional view illustrating a structure of inner tissues of a breast, according to an exemplary embodiment;

FIG. 15 is a flowchart for explaining a method of controlling an X-ray imaging apparatus, according to an exemplary embodiment;

FIG. 16 is a flowchart specifically illustrating a process of acquiring and storing calibration functions of respective pixels for each energy, according to an exemplary embodiment; and

FIG. 17 is a flowchart for explaining a method of controlling an X-ray imaging apparatus, according to an exemplary embodiment.

## DETAILED DESCRIPTION

Reference will now be made in detail to the exemplary embodiments, examples of which are illustrated in the accompanying drawings, wherein like reference numerals refer to like elements throughout.

Hereinafter, exemplary embodiments of an X-ray imaging apparatus and a control method therefor will be described in detail with reference to the accompanying drawings.

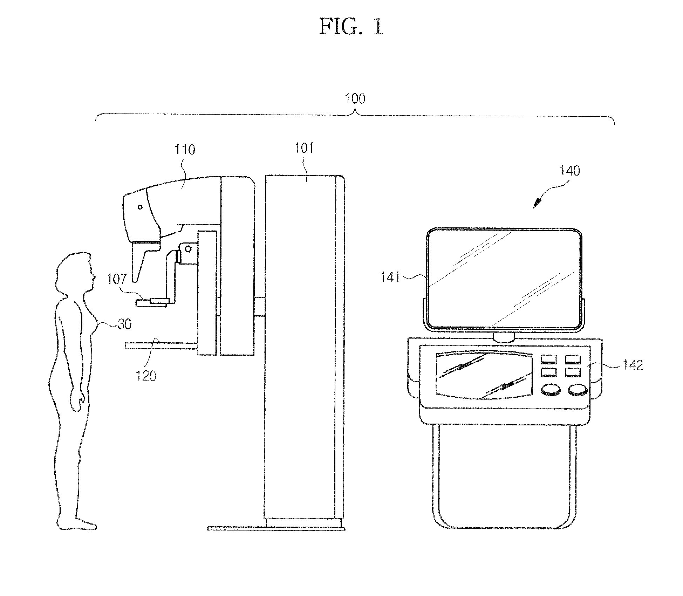

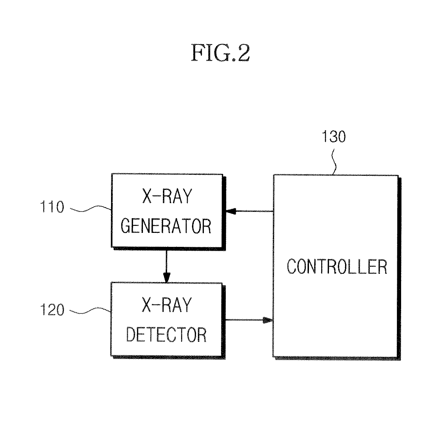

FIG. 1 is a general view of an X-ray imaging apparatus 100 for mammography, according to an exemplary embodiment. FIG. 2 is a control block diagram of the X-ray imaging apparatus 100 according to an exemplary embodiment.

The X-ray imaging apparatus 100 may perform X-ray imaging on various objects and have different structures according to objects to be X-ray imaged. That is, the objects to be X-ray imaged of the X-ray imaging apparatus 100 are not limited. In the present exemplary embodiment, however, the X-ray imaging apparatus 100 for mammography is illustrated.

Referring to FIGS. 1 and 2, the X-ray imaging apparatus 100 includes an X-ray generator 110 to generate X-rays and irradiate an object 30 with the generated X-rays, an X-ray detector 120 to detect X-rays transmitted through the object 30 and convert the detected X-rays into an electrical signal to obtain X-ray data, and a controller 130 that pre-stores calibration functions for correcting errors according to characteristics of each of a plurality of pixels and applies the pre-stored calibration functions to X-ray data obtained when an object is X-ray imaged, thereby correcting an image.

The X-ray generator 110 generates X-rays and irradiates the object 30 with the generated X-rays. When the object 30 is a breast composed of only soft tissues, the object 30 needs to be compressed in a vertical direction to obtain a more clear and accurate image. Thus, the object 30 is positioned between a compression paddle 107 and the X-ray detector 120 and X-rays are irradiated to the object in a state in which the object 30 is compressed. The X-ray generator 110, the X-ray detector 120, and the compression paddle 107 may be supported by a housing 101.

The X-ray generator 110 generates X-rays and irradiates the object 30 with the generated X-rays. The X-ray generator 110 may be supplied with power from a power supply unit (not shown) to generate X-rays. In this regard, energy of the X-rays may be controlled by a tube voltage, and the intensity or dose of the X-rays may be controlled by tube current and X-ray exposure time.

The X-ray generator 110 may irradiate monochromatic X-rays or polychromatic X-rays. In the present exemplary embodiment, however, the X-ray generator 110 irradiates polychromatic X-rays having a certain energy band, and the energy band of the irradiated X-rays is defined by an upper limit and a lower limit.

The upper limit of the energy band, i.e., a maximum energy of the irradiated X-ray, may be controlled by the intensity of the tube voltage, and the lower limit of the energy band, i.e., a minimum energy of the irradiated X-ray, may be controlled by a filter installed inside or outside of the X-ray generator 110. When low-energy band X-ray is filtered through the filter, an average energy of the irradiated X-ray may be increased.

In addition, the X-ray imaging apparatus 100 may include an auto exposure controller (AEC) to control a parameter for X-ray irradiation, for example, at least one parameter of a tube voltage, tube current, a target material of a positive electrode, exposure time, threshold energy, and a filter. The AEC serves to optimize X-ray irradiation conditions to suit an actual object to be X-ray imaged, and may set a parameter optimized to characteristics of the object 30 by analyzing a pre-shot image of the object 30.

The X-ray detector 120 detects X-rays transmitted through the object 30 and converts the detected X-rays into an electrical signal to obtain X-ray data.

In general, an X-ray detector may be classified according to a material composition method, a method of converting detected X-rays into an electrical signal, and a method of acquiring X-ray data.

First, the X-ray detector may be classified into two types according to a material composition method: a monolithic type and a hybrid type.

The monolithic type X-ray detector consists of a semiconductor of a single material which has a region to generate an electrical signal by detecting X-rays and a region to read and process the electrical signal, or is manufactured using a single manufacturing process. For example, a light receiving element such as a charge coupled device (CCD) or a complementary metal oxide semiconductor (CMOS) may be used.

The hybrid type X-ray detector includes a region to generate an electrical signal by detecting X-rays and a region to read and process the electrical signal, which are formed of different materials, or may be manufactured using different manufacturing processes. For example, the hybrid type X-ray detector may include a light receiving element such as photodiodes, CCD, CdZnTe, or the like to detect X-rays and a CMOS read-out integrated circuit (ROIC) to read an electrical signal, may include a strip detector to detect X-rays and a CMOS ROIC to read and process an electrical signal, and may include an a-Si or a-Se flat panel system.

In addition, the X-ray detector may be classified into two types according to a method of converting X-rays into an electrical signal: a direct conversion type and an indirect conversion type.

In the direct conversion type, when X-rays are irradiated, electron-hole pairs are temporarily generated in a light receiving element, and the electrons and holes move towards a positive electrode and a negative electrode, respectively, by an electric field applied between opposite ends of the light receiving element. In this regard, the X-ray detector converts such movement into an electrical signal. The light receiving element of the direction conversion type X-ray detector may include a-Se, CdZnTe, $HgI_2$, $PbI_2$, or the like.

In the indirect conversion type, the X-ray detector includes a scintillator between a light receiving unit and an X-ray generator. When X-ray irradiated from the X-ray generator reacts with the scintillator to emit photons having a visible light wavelength, the light receiving element senses the emitted photons and converts the photons into an electrical signal. In the indirect conversion type, a-Si may be used as a constituent of the light receiving element, and a thin film-type GADOX scintillator, a micro-column type scintillator, or a needle structured type CSI(T1) scintillator may be used as the scintillator.

In addition, the X-ray detector may be classified into two types according to an X-ray data acquisition method: a charge integration mode and a photon counting mode. In the charge integration mode, charges are stored for a certain period of time and a signal is acquired therefrom, and, in the photon counting mode, photons having an energy level that is equal to or higher than threshold energy level are counted whenever a signal is generated by single X-ray photons.

The X-ray imaging apparatus 100 according to an exemplary embodiment uses the photon counting mode which provides a smaller amount of X-ray exposure to an object and less noise in an X-ray image than in the charge integration mode. Thus, the X-ray detector 120 may be a photon counting detector (PCD).

Although the material composition method and the method of conversion into an electrical signal of the X-ray detector 120 are not limited thereto, an exemplary embodiment of the X-ray detector 120 to which a direct conversion method for directly acquiring an electrical signal from X-rays and a hybrid type in which a light receiving element for detecting X-rays and a read-out circuit are coupled are applied will be described in detail for convenience of explanation.

A host device 140 includes a display unit 141 to display a generated X-ray image and an input unit 142 to receive all the commands related to operations of the X-ray imaging apparatus 100 from a user. In addition, the host device 140 may implement a process for forming an X-ray image using X-ray data transmitted from the X-ray detector 120, and this process may be performed in a case in which the controller 130, which will be described below, is included in the host device 140. However, exemplary embodiments are not limited to the above example, and there is no limitation as to disposition of the controller 130 so long as the controller 130 may implement a function, which will be described below.

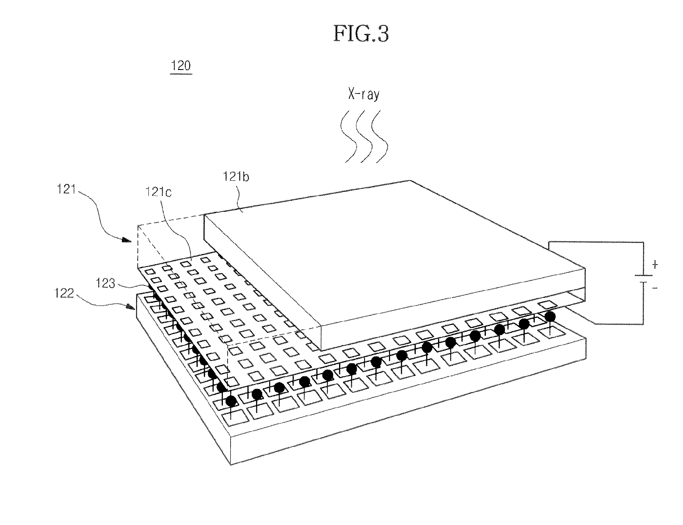

FIG. 3 is a schematic diagram illustrating a structure of the X-ray detector 120 of the X-ray imaging apparatus 100 according to an exemplary embodiment.

Referring to FIG. 3, the X-ray detector 120 includes a light receiving element 121 to detect X-rays and convert the detected X-rays into an electrical signal and a readout circuit 122 to read the electrical signal. In this regard, the readout circuit 122 is in the form of a two-dimensional pixel array including a plurality of pixel regions. The light receiving element 121 may be formed of a monocrystalline semiconductor material to achieve low energy, high resolution at low dose, a quick response time, and a high dynamic region. The monocrystalline semiconductor material may be, for example, Ge, CdTe, CdZnTe, GaAs, or the like.

The light receiving element 121 may be in the form of a PIN photodiode such that a p-type layer 121c in which p-type semiconductors are arranged in a two-dimensional pixel array structure is attached to a lower surface of a high resistance n-type semiconductor substrate 121b, and the readout circuit 122 using a CMOS manufacturing process is coupled with the light receiving element 121 on a per pixel basis. The read-out circuit 122 and the light receiving element 121 may be coupled by a flip-chip bonding method such that bumps 123 formed of solder (PbSn), indium (In), or the like are formed on the read-out circuit 122 and subjected to a reflow process and heated, and the read-out circuit 122 and the light receiving element 121 are pressed against each other. However, the above-described structure of the X-ray detector 120 is only an exemplary embodiment and the structure thereof is not limited thereto.

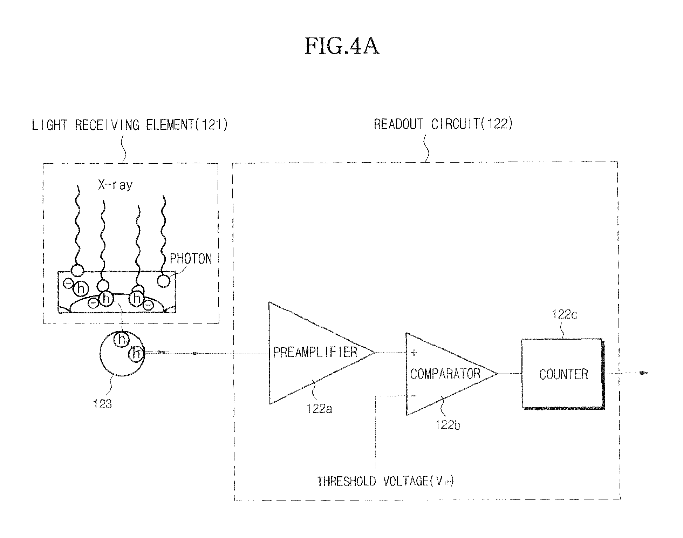

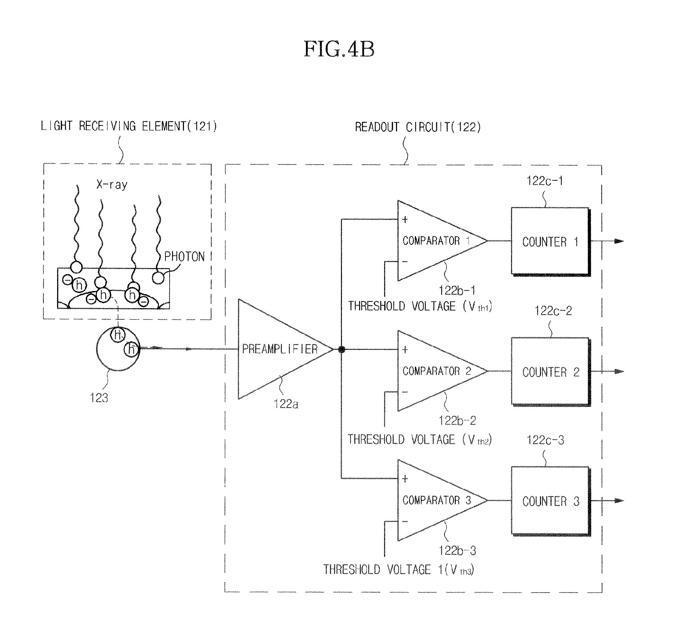

FIG. 4A is a schematic diagram illustrating a structure of a single pixel region of the X-ray detector 120 illustrated in FIG. 3. FIG. 4B is a schematic diagram illustrating a structure of a single pixel region in which detected X-rays are divided according to a plurality of energy bands.

Referring to FIG. 4A, when photons of X-rays are incident upon the light receiving element 121, electrons in a valence band receive energy of the photons which is equal to or greater than a band-gap energy difference and are excited to a conduction band. Accordingly, electron-hole pairs are generated in a depletion region.

A metal electrode is formed at each of the p-type layer 121c and the n-type semiconductor substrate 121b of the light receiving element 121. When a reverse bias is applied between the metal electrodes, electrons of the electron-hole pairs generated in the depletion region are attracted toward an n-type region and holes thereof are attracted toward a p-type region. The holes attracted toward a p-type region are input to the read-out circuit 122 via the bumps 123 so that an electrical signal generated by photons is read. However, electrons may be input to the read-out circuit 122 according to the structure of the light receiving element 121 and an applied voltage so that an electrical signal is generated.

The read-out circuit 122 may take the form of a two-dimensional pixel array corresponding to the p-type semiconductors of the light receiving element 121, and each pixel of the read-out circuit 122 reads out an electrical signal. When charges are input to the read-out circuit 122 from the light receiving element 121 via the bumps 123, a preamplifier 122a of the readout circuit 122 is charged with an input charge generated from a single photon and outputs a voltage signal corresponding thereto.

The voltage signal output from the preamplifier 122a is transmitted to a comparator 122b, the comparator 122b compares an externally controllable threshold voltage with the input voltage signal to output a pulse signal of '1' or '0' according to comparison results, and a counter 122c counts the output number of '1' and outputs digitized X-ray data. X-ray data according to each pixel may be combined to acquire an X-ray image.

In this regard, a threshold voltage corresponds to threshold energy level E. When counting the number of photons having an energy level that is equal to or greater than E, a threshold voltage corresponding to the threshold energy level E is input to the comparator 122b. The threshold voltage and the threshold energy E level may correspond to each other because the amplitude of the electrical signal (voltage) generated by the light receiving element 121 varies according to energy level of photons. Thus, a desired threshold voltage corresponding to threshold energy level may be calculated using an equation showing a relationship between energy level of photons and generated voltage. Therefore, in the following exemplary embodiments, the expression "threshold energy level is input to the X-ray detector 120" is intended to mean that a threshold voltage corresponding to the threshold energy level is input thereto.

To improve a contrast between inner materials of the object 30, X-ray images having a plurality of different energy bands may be acquired to form a multiple energy X-ray image. In this regard, to acquire the X-ray images having a plurality of different energy bands, X-rays may be irradiated a plurality of times by varying energy bands of the X-ray. In the present embodiment, however, a PCD is used as the X-ray detector 120 of the X-ray imaging apparatus 100 and thus the X-ray generator 110 irradiates broadband X-rays having a plurality of energy bands once and the X-ray detector 120 sorts the detected X-rays according to the plurality of energy bands.

For this operation, as illustrated in FIG. 4B, a plurality of comparators (i.e., comparators 1, 2 and 3 122b-1, 122b-2 and 122b-3 and a plurality of counters (i.e., counters 1, 2 and 3 122c-1, 122c-2 and 122c-3) are installed to count photons according to a plurality of energy bands. Although the number of the comparators illustrated in FIG. 4B is three, exemplary embodiments are not limited thereto. That is, the number of the comparators may be determined by the number of energy bands to be divided.

Referring to FIG. 4B, when electrons or holes generated by a single photon are input to the preamplifier 122a, the electrons or holes are output as a voltage signal and the voltage signal is input to the comparators 1, 2 and 3 122b-1, 122b-2 and 122b-3. When threshold voltages 1, 2 and 3 $V_{th1}$, $V_{th2}$ and $V_{th3}$ are respectively input to the comparators 1, 2 and 3 122b-1, 122b-2 and 122b-3, the comparator 1 122b-1 compares the input voltage with the threshold voltage 1 $V_{th1}$ and the counter 1 122c-1 counts the number of photons generating a voltage that is greater than the threshold voltage 1 $V_{th1}$. In the same manner, the counter 2 122c-2 counts the number of photons generating a voltage that is greater than the threshold voltage 2 $V_{th2}$, and the counter 3 122c-3 counts the number of photons generating a voltage that is greater than the threshold voltage 3 $V_{th3}$.

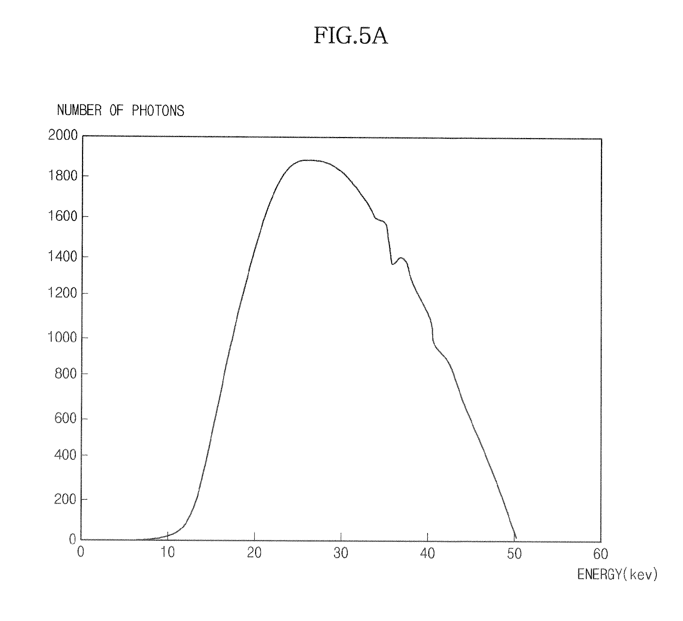

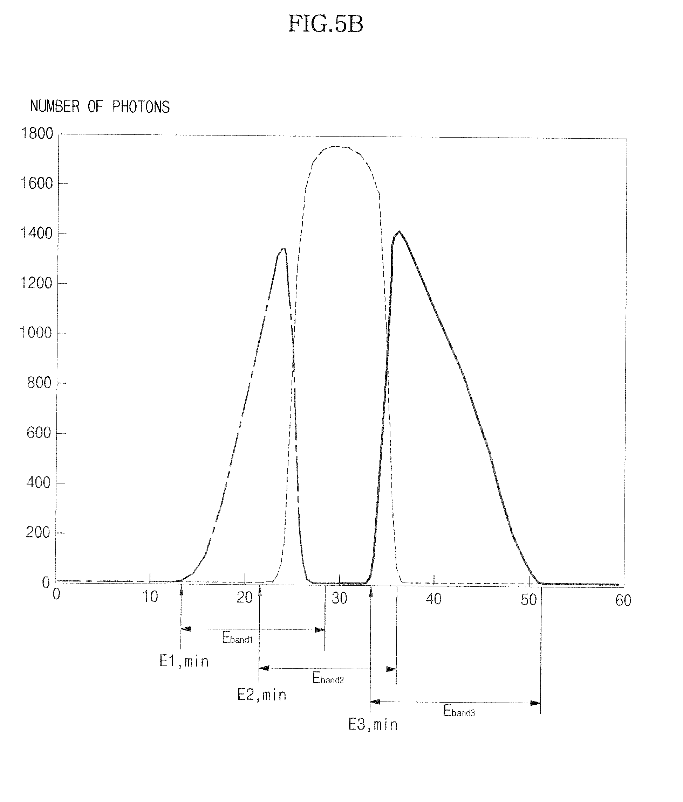

FIG. 5A is a graph showing an energy spectrum of X-rays irradiated by an X-ray generator, according to an exemplary embodiment. FIG. 5B is a graph showing an ideal spectrum for a case in which the X-ray detector of FIG. 4A divides X-rays according to energy bands, according to an exemplary embodiment.

The energy of X-rays irradiated by the X-ray generator 110 is adaptively varied to suit an object. Thus, when a breast is imaged, as illustrated in FIG. 5A, the X-ray generator 110 may generate X-rays having an energy lower limit of 10 keV and an energy upper limit of 50 keV and irradiate the object with the generated X-rays. For this, the X-rays may be generated at a tube voltage of 50 kVp (50 kV) and irradiated after filtering a low energy band thereof (i.e., about 0 to about 10 keV). In this regard, an X-ray dose (number of photons) represented by the y axis of a graph illustrated in FIG. 5A may be controlled by tube current and X-ray exposure time.

X-rays detected by the X-ray detector 120 may be divided according to three energy bands $E_{band1}$, $E_{band2}$ and $E_{band3}$, as illustrated in FIG. 5B. For this operation, a voltage corresponding to $E_{1,min}$ may be calculated and input as a threshold voltage to the comparator 1 122b-1 of FIG. 4B, a voltage corresponding to $E_{2,min}$ may be calculated and input as a threshold voltage to the comparator 2 122b-2, and a voltage corresponding to $E_{3,min}$ may be calculated and input as a threshold voltage to the comparator 3 122b-3.

Theoretically, the amplitude of a voltage signal generated by each pixel of the X-ray detector 120 is affected only by energy of irradiated photons, but may also be affected by characteristics of the light receiving element 121 or the read-out circuit 122 of each pixel. Thus, even though photons having the same energy are incident upon all the pixels, the amplitude of a voltage signal generated by a single photon may vary according to characteristics of each pixel.

In particular, when errors are present in a signal that is input to or output from the preamplifier 122a because characteristics of the light receiving element 121 or the preamplifier 122a of each pixel are different, errors may occur in X-ray data output from a counter.

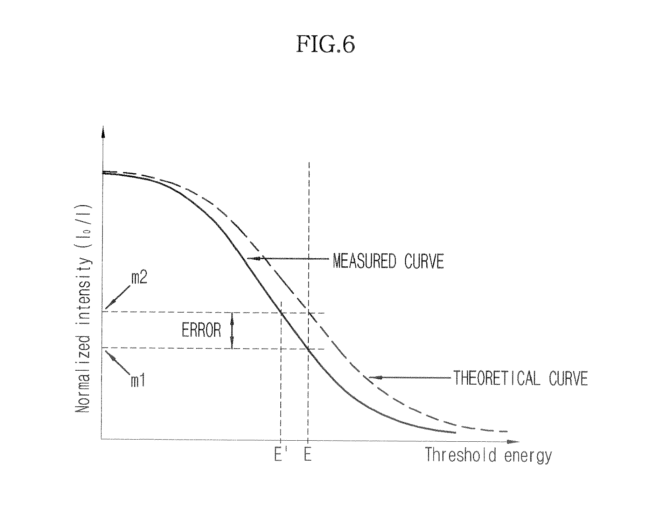

FIG. 6 is a graph showing a plot of a measured X-ray intensity normalized for each threshold energy level and a theoretical plot thereof, according to an exemplary embodiment.

Referring to FIG. 6, errors may occur between a curve of normalized X-ray intensity measured by varying threshold energy level and a theoretical curve of normalized X-ray intensity under the same conditions according to characteristics of pixels.

For example, when a value of threshold energy level to be input is E, that is, when the number of photons having an energy level that is equal to or greater than E is counted, theoretical normalized X-ray intensity with respect to a threshold energy level of E is $m_2$. When a threshold energy level of E is actually input, however, measured normalized X-ray intensity may be $m_1$.

By comparing the curve of measured normalized X-ray intensity with the theoretical curve of normalized X-ray intensity, it can be confirmed that a threshold energy level of E' needs to be input to acquire the threshold normalized X-ray intensity of $m_2$ corresponding to a threshold energy level of E or higher under the same conditions through the X-ray detector 120.

The X-ray imaging apparatus 100 may correct threshold energy level according to characteristics of each pixel by changing a structure of the X-ray detector 120 in a hardware manner. To minimize design complexity, however, the correcting process may not be used, and, even though threshold energy level is corrected according to each pixel, accurate correction may not be implemented due to the limitation on the number of bits assigned to each pixel.

Thus, the controller 130 estimates in advance, a calibration function for each pixel to correct X-ray data using data measured for phantoms, and applies the calibration function for each pixel to X-ray data acquired by performing X-ray imaging on an actual object, thereby correcting an X-ray image of the object.

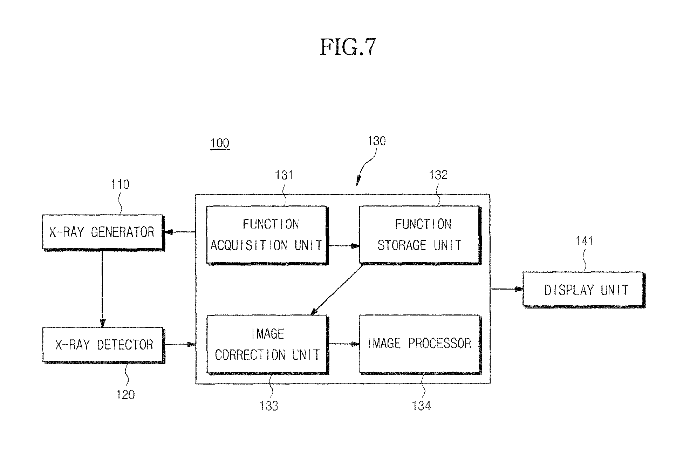

FIG. 7 is a control block diagram specifically illustrating a structure of the controller 130 of the X-ray imaging apparatus 100 according to an exemplary embodiment.

Referring to FIG. 7, the controller 130 includes a function acquisition unit 131 to acquire, e.g., determine, a calibration function for each pixel, a function storage unit 132 to store the acquired calibration function, an image correction unit 133 to apply the calibration function for each pixel to X-ray data acquired by performing X-ray imaging on an actual object, and an image processor 134 to perform image processing for image enhancement of the corrected image and output the enhanced image via the display unit 140.

The function acquisition unit 131 acquires, i.e., calculates, a calibration function for correcting X-ray data output from the X-ray detector 120. The acquisition of the calibration function may be performed before X-ray imaging of an actual object, may be performed once or periodically before use of the X-ray imaging apparatus 100, or may be performed whenever components of the X-ray imaging apparatus 100 are replaced or without a predetermined cycle. In the X-ray imaging apparatus 100, the acquisition time or number of the calibration function is not limited.

The function storage unit 132 stores the calibration functions acquired by the function acquisition unit 131, for each pixel and each threshold energy level to be used for the correction of an image of an actual object. Hereinafter, operations of the function acquisition unit 131 and the function storage unit 132 will be described in detail.

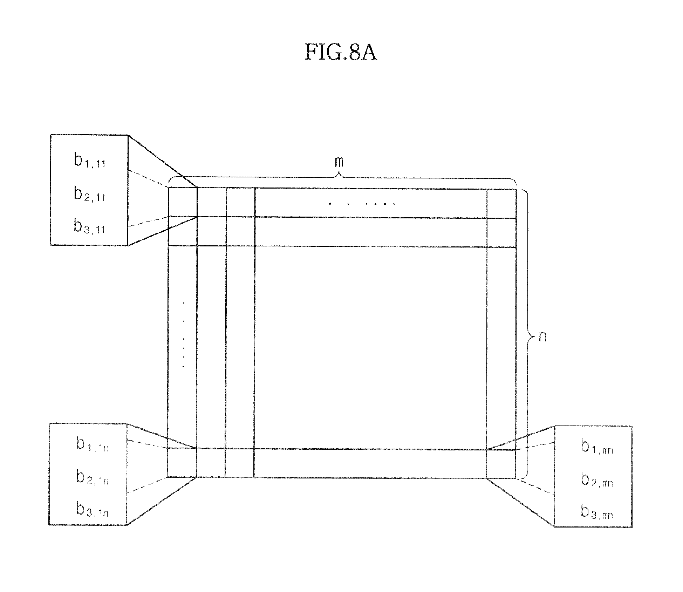

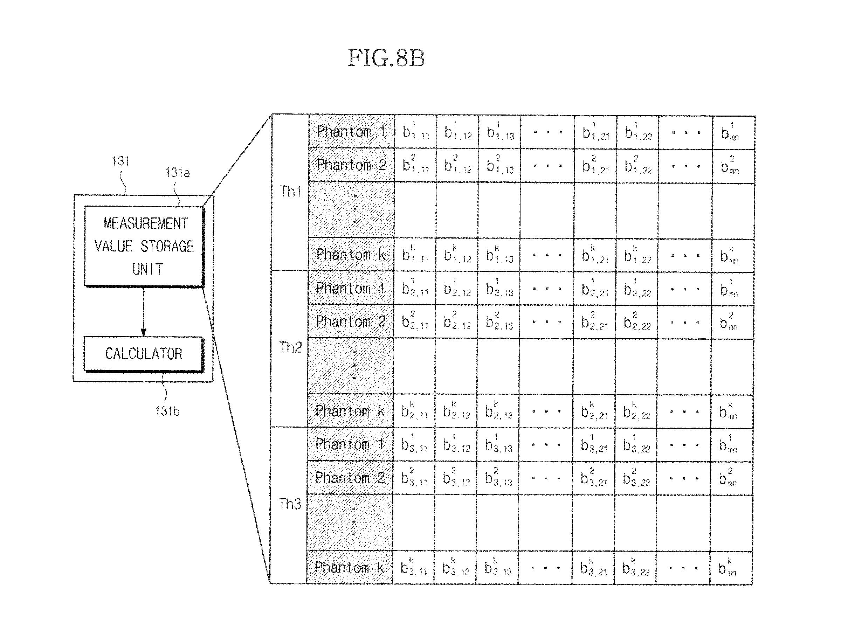

FIG. 8A is a schematic view of X-ray data output from each pixel of the X-ray detector 120 of the X-ray imaging apparatus 100 according to an exemplary embodiment. FIG. 8B is a schematic view of data stored by the function acquisition unit 131 of the X-ray imaging apparatus 100 according to an exemplary embodiment.

X-ray data is data output from each pixel and refers to the number of photons having an energy level that is equal to or greater than threshold energy level among photons input to the pixels. For example, when three threshold energy level values are input to each pixel of the X-ray detector 120 and the X-ray detector 120 consists of m*n pixels where m and n are each independently a natural number, as illustrated in FIG. 8A, the m*n pixels $PX_{11}$, $PX_{12}$, ....., and $PX_{mn}$ respectively output data corresponding to threshold energy 1 $b_{1,11}$, $b_{1,12}$,....., and $b_{1,mn}$, data corresponding to threshold energy 2 $b_{2,11}$, $b_{2,12}$, ....., and $b_{2,mn}$, and data corresponding to threshold energy 3 $b_{3,11}$, $b_{3,12}$, ....., and $b_{3,mn}$. In this regard, the subscripts of PX and second subscripts of b denote coordinates representing the position of pixels corresponding to the corresponding data in the two-dimensional pixel array, and the first subscripts of b denote the corresponding threshold energy level.

Referring to FIG. 8B, the function acquisition unit 131 includes a measurement value storage unit 131a to store measurement values for predesigned phantoms and a calculator 131b to perform calculation to acquire a calibration function using the stored measurement values.

The phantoms may cover all the pixels of the X-ray detector 120 and be designed to have different thicknesses and material compositions, each of which has uniform thickness and material composition with respect to all the pixel regions.

Radiography is performed on the designed phantoms under radiography conditions of an actual object to measure X-ray data. In this regard, the radiography may be performed for sufficient X-ray exposure time to minimize impact of quantum noise. The radiography conditions of an actual object include at least one of a tube voltage and tube current of the X-ray generator 110, a target material of a positive electrode, exposure time, the type of a filter, and threshold energy level input to the X-ray detector 120.

The measured X-ray data is stored in the measurement value storage unit 131a according to threshold energy level 1 $Th_1$, threshold energy level 2 $Th_2$, and threshold energy level 3 $Th_3$ and the types of the phantoms $phantom_1$ through $phantom_k$. When the number of radiographed phantoms is k where k is a natural number, X-ray data corresponding to each threshold energy level (e.g., $b_{11}$, $b_{12}$,....., and $b_{mn}$ corresponding to threshold energy level 1 $Th_1$) are stored according to the types of the phantoms $phantom_1$ through $phantom_k$. In FIG. 8B, the superscripts of the X-ray data denote the types of the phantoms, the first subscripts of the X-ray data denote threshold energy level, and the second subscripts of the X-ray data denote the positions of the corresponding pixels.

The calculator 131b performs calculation to acquire calibration functions using the data stored in the measurement value storage unit 131a. Errors in output values of the X-ray detector 120 are derived from the characteristics of the light receiving element 121 or the characteristics of the read-out circuit 122, and thus the calibration function is acquired for each pixel.

In addition, as illustrated in FIG. 4B, when the plurality of comparators and counters are installed to divide the detected X-rays according to a plurality of energy bands, characteristics of each of the comparators and counters may be different. In this case, a calibration function of each pixel is acquired for each threshold energy level.

In an exemplary embodiment, the calculator 131b may determine a coefficient of a polynomial using the stored data of the measurement value storage unit 131a, assuming that the calibration function is a polynomial defined by a plurality of coefficients. To acquire calibration functions for a single threshold energy, X-ray data corresponding to another threshold energy level may be used, and the calibration functions may be defined by Equation 1 through Equation 3 below:

[Equation 1]

$$b_1' = C_{1,0} + C_{1,1}b_1 + C_{1,2}b_2 + C_{1,3}b_3 + C_{1,4}b_1b_2 + C_{1,5}b_1b_3 + C_{1,6}b_2b_3 + C_{1,7}b_1{}^2 + C_{1,8}b_2{}^2 + C_{1,9}b_3{}^2$$

[Equation 2]

$$b_2' = C_{2,0} + C_{2,1}b_1 + C_{2,2}b_2 + C_{2,3}b_3 + C_{2,4}b_1b_2 + C_{2,5}b_1b_3 + C_{2,6}b_2b_3 + C_{2,7}b_1{}^2 + C_{2,8}b_2{}^2 + C_{2,9}b_3{}^2$$

[Equation 3]

$$b_3' = C_{3,0} + C_{3,1}b_1 + C_{3,2}b_2 + C_{3,3}b_3 + C_{3,4}b_1b_2 + C_{3,5}b_1b_3 + C_{3,6}b_2b_3 + C_{3,7}b_1{}^2 + C_{3,8}b_2{}^2 + C_{3,9}b_3{}^2.$$

In Equation 1 through Equation 3, $b_1'$, $b_2'$, and $b_3'$ are ideal X-ray data corresponding to threshold energy level 1 $Th_1$, threshold energy level 2 $Th_2$, and threshold energy level 3 $Th_3$, respectively, i.e., X-ray data to which errors according to characteristics of each pixel are not applied. In addition, $b_1$, $b_2$, and $b_3$ are X-ray data for each threshold energy level stored in the measurement value storage unit 131a, and C is the coefficient of the polynomial. Thus, Equation 1, Equation 2, and Equation 3 are functions representing the relationship between the measured X-ray data and ideal X-ray data.

Calculation for the acquisition of the calibration function is performed on a per pixel basis for each threshold energy level. First, an operation of the calculator 131b to acquire a calibration function corresponding to a pixel having a coordinate of (1,1) (hereinafter, referred to as "$PX_{11}$") and threshold energy level 1 $Th_1$ will be described.

The polynomial of Equation 1 includes 10 coefficients, and thus 10 equations are needed to determine the 10 unidentified coefficients. Thus, X-ray data measured by irradiating X-rays to at least 10 phantoms are stored in the measurement value storage unit 131a. However, exemplary embodiments are not limited to the above example, the number of the coefficients is not limited, and the number of the coefficients may be appropriately set, as desired. In addition, the number of the phantoms varies according to the number of the coefficients.

Referring to FIG. 8B, data needed to acquire the calibration function corresponding to $Pixel_{11}$ and threshold energy level 1 $Th_1$ is a data set in the first row of a data structure illustrated in FIG. 8B. In particular, the calculator 131b makes an equation for phantom 1 by substituting $b^1{}_{1,11}$ for $b_1$ of Equation 1, $b^1{}_{2,11}$ for $b_2$ of Equation 1, and $b^1{}_{3,11}$ for $b_3$ of Equation 1, and the data set in the first row of a data structure of FIG. 8B, i.e., a representative value of the data set corresponding to threshold energy level 1 and phantom 1, may be substituted for ideal data $b_1$'. In this regard, the representative value may be at least one selected from the group consisting of the most frequent value among m*n pixel values, a median of the m*n pixel values, and an average of a weighted sum obtained by applying an appropriate weight to each pixel value and the m*n pixel values (where m and n are each independently a natural number). The representative value may be stored in the measurement value storage unit 131a together with the measured X-ray data.

In the same manner, the calculator 131b makes an equation for each of the remaining nine phantoms, to complete formation of 10 equations. Then, the calculator 131b solves the 10 equations to determine values for the 10 coefficients. The determined coefficient value is substituted for the 10 coefficients of Equation 1 and thus the calibration function corresponding to $PX_{11}$ and threshold energy level 1 $Th_1$ is acquired.

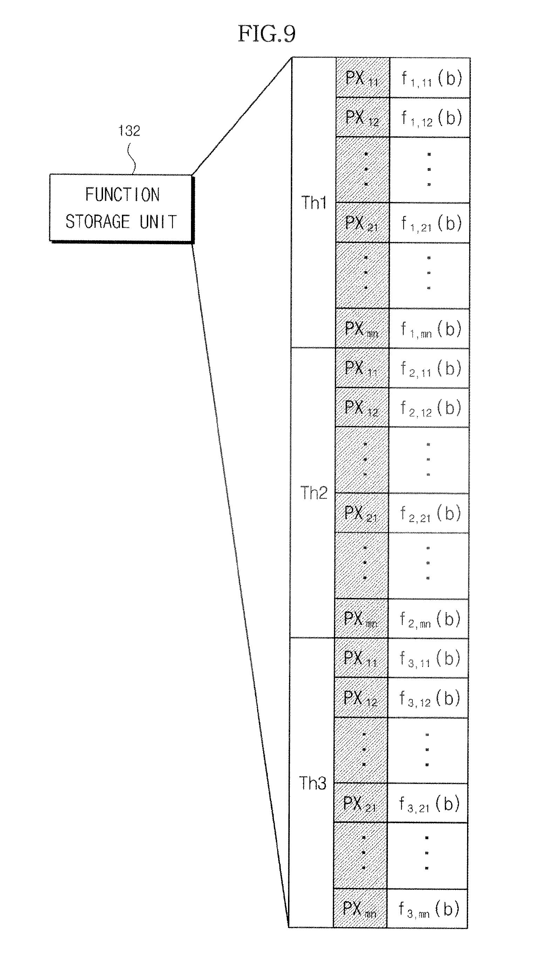

FIG. 9 is a schematic view of a structure of data stored in the function storage unit 132, according to an exemplary embodiment.

The calculator 131b may acquire calibration functions of respective pixels for each threshold energy level using the same method as that used to acquire the calibration function corresponding to threshold energy level 1 $Th_1$ and $PX_{11}$, and the acquired calibration functions are stored in the function storage unit 132.

Referring to FIG. 9, a function $f_{1,11}$ for threshold energy level 1 $Th_1$ and $PX_{11}$ through a function $f_{3,mn}$ for threshold energy level 3 $Th_3$ and $PX_{mn}$ may be stored in the function storage unit 132 for each pixel and each threshold energy level. Thus, when an image of an actual object is corrected, the functions stored in the function storage unit 132 may be called and used.

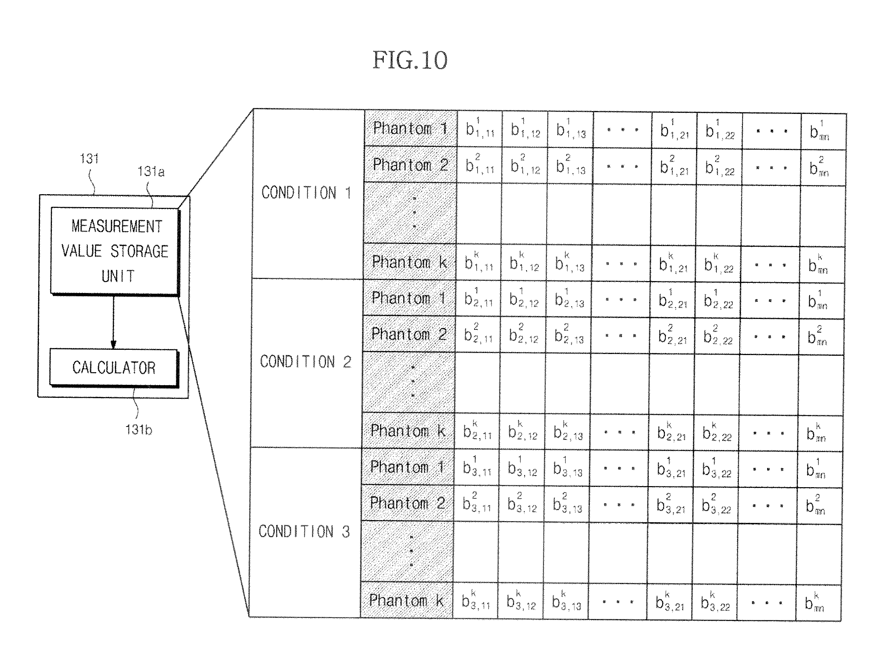

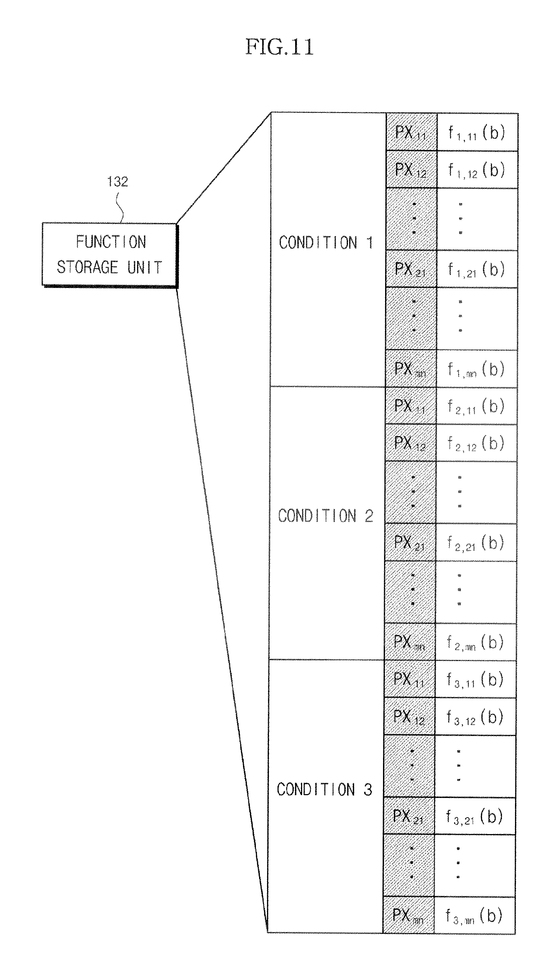

FIG. 10 is a schematic view of a structure of data stored in the measurement value storage unit 131a when calibration functions are acquired for each condition, according to an exemplary embodiment. FIG. 11 is a schematic view of a structure of data stored in the function storage unit 132 when calibration functions are acquired for each condition, according to an exemplary embodiment.

As described above, the X-ray imaging apparatus 100 may include an AEC to optimize radiography conditions for an object. In this case, the radiography conditions may vary according to objects, and thus the X-ray imaging apparatus 100 may acquire calibration functions according to radiography conditions.

Referring to FIG. 10, several sets of radiography conditions that may be set by the AEC are previously assumed, X-rays are irradiated to phantoms according to the radiography condition sets to measure X-ray data, and the measured X-ray data are stored in the measurement value storage unit 131a. The above-described radiography conditions such as a tube voltage, tube current, a target material of a positive electrode, exposure time, threshold energy level, the type of a filter, and the like may constitute a radiography condition set. In the present exemplary embodiment, X-ray data for three radiography condition sets are measured, and a single threshold energy level is input to each pixel. Thus, the X-ray data measured under conditions 1, 2 and 3 are stored in the measurement value storage unit 131a, for each phantom and each pixel.

The calculator 131b performs calculation using the same method as described above to acquire calibration functions, except that Equation 1 corresponds to condition 1 instead of threshold energy level 1 $Th_1$, Equation 2 corresponds to condition 2, and Equation 3 corresponds to condition 3.

As illustrated in FIG. 11, the acquired calibration functions are stored in the function storage unit 132 for conditions 1, 2 and 3 and pixels $PX_{11}$ through $PX_{mn}$, and, when an image of an actual object is corrected, functions corresponding to conditions applied to radiography for the object may be called from the stored calibration functions and used.

Referring back to FIG. 7, the image correction unit 133 corrects an X-ray image obtained by radiography of an actual object using the calibration functions stored in the function storage unit 132.

In a case in which the exemplary embodiments illustrated in FIGS. 8 and 9 are applied assuming that the X-ray detector 120 divides X-rays input to pixels according to three threshold energy levels, first, the X-ray generator 110 irradiates an object with broadband X-rays having three energy bands, and the X-ray detector 120 detects the X-rays transmitted through the object to output the number of photons having an energy level that is greater than threshold energy level 1 $Th_1$, the number of photons having an energy level that is greater than threshold energy level 2 $Th_2$, and the number of photons having an energy level that is greater than threshold energy level 3 $Th_3$ as X-ray data corresponding to threshold energy level 1 $Th_1$, X-ray data corresponding to threshold energy level 2 $Th_2$, and X-ray data corresponding to threshold energy level 3 $Th_3$, respectively.

The output X-ray data are input to the image correction unit 133 and corrected. The X-ray data corresponding to threshold energy level 1 $Th_1$ form an X-ray image corresponding to threshold energy level 1 $Th_1$, the X-ray data corresponding to threshold energy level 2 $Th_2$ form an X-ray image corresponding to threshold energy level 2 $Th_2$, and X-ray data corresponding to threshold energy level 3 $Th_3$ form an X-ray image corresponding to threshold energy level 3 $Th_3$. Thus, correction of the X-ray data by the image correction unit 133 may mean correction of X-ray images.

The image correction unit 133 calls functions $f_{1,11}$ through $f_{1,mn}$ corresponding to threshold energy level 1 $Th_1$ from the function storage unit 132 to correct the X-ray image corresponding to threshold energy level 1 $Th_1$. X-ray data of each pixel are corrected using a function corresponding to the corresponding pixel.

For example, X-ray data of $PX_{11}$ are corrected using the function $f_{1,11}$. When the function acquisition unit 131 acquires a calibration function using Equation 1, the function $f_{1,11}$ corresponding to threshold energy level 1 $Th_1$ may be represented by Equation 4.

[Equation 4]

$$f_{1,11}(b_1, b_2, b_3) = C_{1,0} + C_{1,1}b_1 + C_{1,2}b_2 + C_{1,3}b_3 + C_{1,4}b_1b_2 + C_{1,5}b_1b_3 + C_{1,6}b_2b_3$$

$$+ C_{1,7}b_1{}^2 + C_{1,8}b_2{}^2 + C_{1,9}b_3{}^2$$

In Equation 4, $C_{1,0}$ through $C_{1,9}$ are predetermined coefficients, and $b_1$, $b_2$, and $b_3$ are variables. In this regard, when X-ray data ($b_{1,11}$ , $b_{2,11}$ , $b_{3,11}$) corresponding to each threshold energy level output from $PX_{11}$ are substituted for $b_1$, $b_2$, and $b_3$, error-corrected X-ray data may be obtained. When X-ray data are corrected in the same manner as described above for the remaining pixels, the X-ray image corresponding to threshold energy level 1 $Th_1$ is corrected.

The image correction unit 133 calls functions $f_{2,11}$ through $f_{2,mn}$ corresponding to threshold energy level 2 $Th_2$ from the function storage unit 132 to correct an X-ray image corresponding to threshold energy level 2 $Th_2$. X-ray data of each pixel are corrected using a function corresponding to the corresponding pixel.

For example, X-ray data of $PX_{11}$ are corrected using the function $f_{2,11}$. When the function acquisition unit 131 acquires a calibration function using Equation 2, the function $f_{2,11}$ corresponding to threshold energy level 2 $Th_2$ may be represented by Equation 5.

[Equation 5]

$$f_{2,11}(b_1, b_2, b_3) = C_{2,0} + C_{2,1}b_1 + C_{2,2}b_2 + C_{2,3}b_3 + C_{2,4}b_1b_2 + C_{2,5}b_1b_3 + C_{2,6}b_2b_3$$

$$+ C_{2,7}b_1{}^2 + C_{2,8}b_2{}^2 + C_{2,9}b_3{}^2$$

In Equation 5, $C_{2,0}$ through $C_{2,9}$ are predetermined coefficients, and $b_1$, $b_2$, and $b_3$ are variables. In this regard, when X-ray data ($b_{1,11}$ , $b_{2,11}$ , $b_{3,11}$) corresponding to each threshold energy level output from $PX_{11}$ are substituted for $b_1$, $b_2$, and $b_3$, error-corrected X-ray data may be obtained. When X-ray data are corrected in the same manner as described above for the remaining pixels, the X-ray image corresponding to threshold energy level 2 $Th_2$ is corrected.

The image correction unit 133 calls functions $f_{3,11}$ through $f_{3,mn}$ corresponding to threshold energy level 3 $Th_3$ from the function storage unit 132 to correct an X-ray image corresponding to threshold energy level 3 $Th_3$. X-ray data of each pixel are corrected using a function corresponding to the corresponding pixel.

For example, X-ray data of $PX_{11}$ are corrected using the function $f_{3,11}$. When the function acquisition unit 131 acquires a calibration function using Equation 1, the function $f_{3,11}$ corresponding to threshold energy level 3 $Th_3$ may be represented by Equation 6.

[Equation 6]

$$f_{3,11}(b_1, b_2, b_3) = C_{3,0} + C_{3,1}b_1 + C_{13,2}b_2 + C_{3,3}b_3 + C_{3,4}b_1b_2 + C_{3,5}b_1b_3 + C_{3,6}b_2b_3$$

$$+ C_{3,7}b_1{}^2 + C_{3,8}b_2{}^2 + C_{3,9}b_3{}^2$$

In Equation 6, $C_{3,0}$ through $C_{3,9}$ are predetermined coefficients, and $b_1$, $b_2$, and $b_3$ are variables. In this regard, when X-ray data ($b_{1,11}$ , $b_{2,11}$ , $b_{3,11}$) corresponding to each threshold energy level output from $PX_{11}$ are substituted for $b_1$, $b_2$, and $b_3$, error-corrected X-ray data may be obtained. When X-ray data are corrected in the same manner as described above for the remaining pixels, the X-ray image corresponding to threshold energy level 3 $Th_3$ is corrected.

The image processor 134 performs image processing on the corrected X-ray image. The image processing includes at least one of image processing for image quality improvement or image enhancement and image processing for the acquisition of a multiple-energy X-ray image. When the image processor 134 performs the latter image processing, X-rays detected by the X-ray detector 120 are divided according to a plurality of energy bands.

In particular, in a case in which an object 30 is a breast and the X-ray detector 120 outputs X-ray images corresponding to three threshold energy levels, the image correction unit 133 corrects the X-ray images, and the image processor 134 acquires X-ray images according to energy bands from the corrected X-ray images and may acquire X-ray images of three kinds of soft tissues having different attenuation characteristics by subtraction by multiplying the X-ray images according to energy bands by an appropriate weight.

According to another exemplary embodiment, when an object is a chest and the X-ray detector 120 outputs X-ray images corresponding to two threshold energies, the image correction unit 133 corrects the X-ray images, and the image processor 134 acquires X-ray images according to energy bands from the corrected X-ray images and may acquire X-ray images of bones and soft tissues by subtraction by multiplying the X-ray images according to energy bands by an appropriate weight.

The final X-ray image output from the image processor 134 is displayed on the display unit 141.

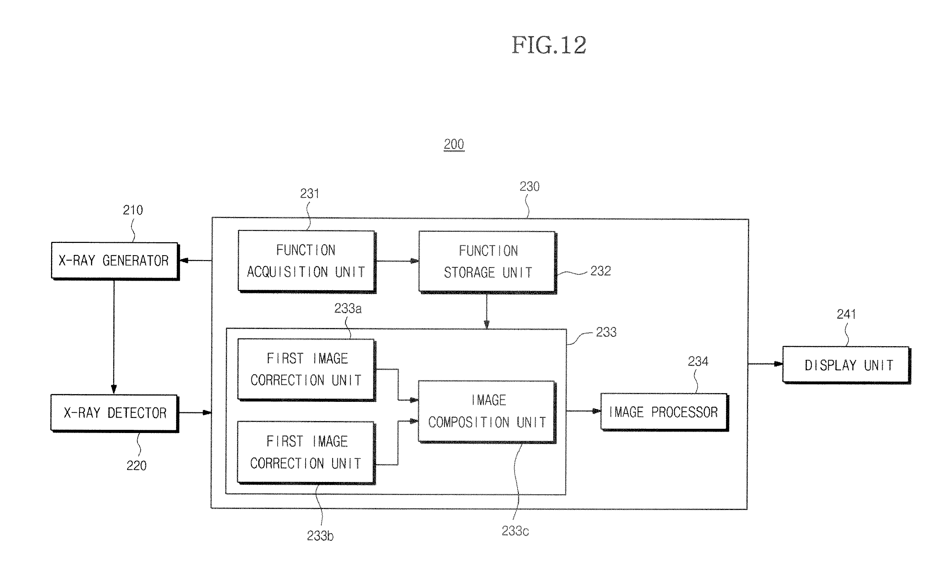

FIG. 12 is a control block diagram of an X-ray imaging apparatus 200 according to another exemplary embodiment.

Referring to FIG. 12, the X-ray imaging apparatus 200 includes an X-ray generator 210 to generate X-rays and irradiate an object with the generated X-rays, an X-ray detector 220 to detect X-rays transmitted through the object and convert the detected X-rays into an electrical signal to obtain X-ray data, a controller 230 to acquire calibration functions for correcting errors according to characteristics of each pixel, for each phantom set to pre-store the acquired calibration functions, and to correct an image by applying the pre-stored calibration functions for each phantom set to an X-ray image of an actual object, and a display unit 241 to display the X-ray image.

Configuration of the X-ray generator 210 and the X-ray detector 220 is the same as described in the previous exemplary embodiment and thus a detailed description thereof is omitted.

The controller 230 includes a function acquisition unit 231 to acquire calibration functions for each threshold energy level, each pixel, and each phantom set, a function storage unit 232 to store the acquired calibration functions, an image correction unit 233 to correct an X-ray image of an actual object using the calibration functions, and an image processor 234 to perform image processing on the corrected X-ray image.

The function acquisition unit 231 and the function storage unit 232 acquire calibration functions and store the calibration functions using a similar method to that used in the function acquisition unit 131 and the function storage unit 132 according to the above-described embodiment. In particular, the function acquisition unit 231 acquires calibration functions for at least two phantom sets obtained by dividing a plurality of phantoms into two groups, and the function storage unit 232 stores the acquired calibration functions.

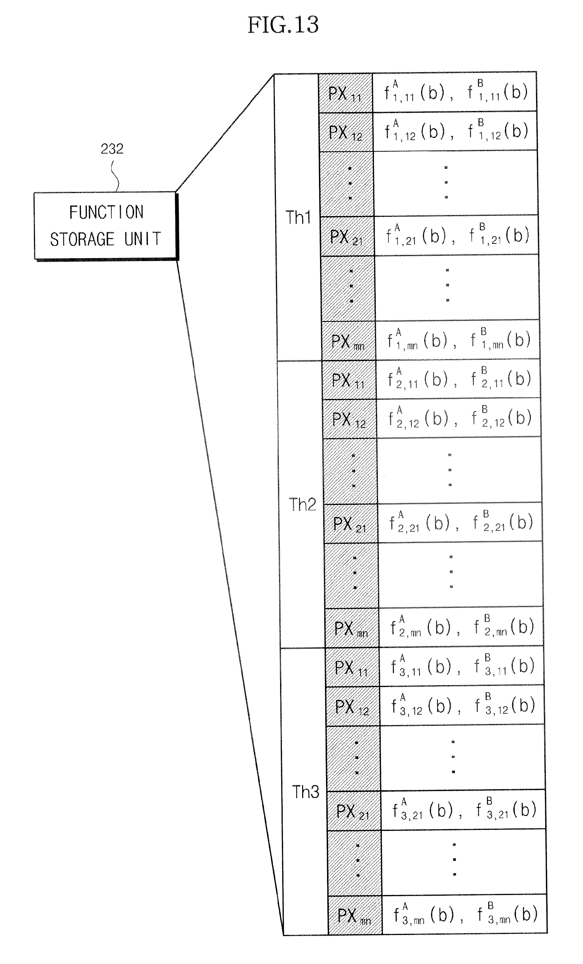

FIG. 13 is a schematic view illustrating a structure of data stored in the function storage unit 232 of the X-ray imaging apparatus 200 according to the exemplary embodiment illustrated in FIG. 12.

For example, in a case in which calibration functions are acquired using Equation 1 through Equation 3 above, X-ray data are measured for 10 types of phantoms belonging to set A and 10 types of phantoms belonging to set B, and calibration functions for the set A and set B are acquired for each threshold energy and each pixel using the measured X-ray data.

Accordingly, as illustrated in FIG. 13, the function storage unit 232 stores a calibration function $f^A(b)$ for the set A and a calibration function $f^B(b)$ for the set B, for threshold energy level 1 $Th_1$, threshold energy level 2 $Th_2$, and threshold energy level 3 $Th_3$ and pixels $PX_{11}$ through $PX_{mn}$. In particular, the function storage unit 232 stores $f^A{}_{1,11}$ and $f^B{}_{1,11}$ corresponding to threshold energy level 1 $Th_1$ and $PX_{11}$, and a method of storing calibration functions for the remaining pixels and threshold energy levels is performed in the same manner as described above.

The image correction unit 233 includes a first image correction unit 233a to correct an X-ray image of an actual object using the calibration function $f^A(b)$ for the set A, a second image correction unit 233b to correct an X-ray image of the actual object using the calibration function $f^B(b)$ for the set B, and an image composition unit 233c to compose the X-ray image corrected using the calibration function $f^A(b)$ for the set A and the X-ray image corrected using the calibration function $f^B(b)$ for the set B.

An X-ray image correction method of the first image correction unit 233a and the second image correction unit 233b is the same as that of the image correction unit 133 according to the previous exemplary embodiment, except that the first image correction unit 233a outputs corrected image A using the calibration function $f^A(b)$ for the set A, and the second image correction unit 233b outputs corrected image B using the calibration function $f^B(b)$ for the set B.

The corrected images A and B are input to the image composition unit 233c, and the image composition unit 233c composes the corrected images A and B, which are obtained using the different calibration functions, to form a corrected final image. When the number of threshold energy levels is 2, the first and second image correction units 233a and 233b correct X-ray images using calibration functions $f^A(b)$ and $f^B(b)$ for an X-ray image corresponding to each threshold energy level, and the image composition unit 233c composes corrected image A and corrected image B for each threshold energy level to form a corrected final image for each threshold energy level.

The image composition unit 233c may perform a composition by comparing the corrected images A and B with each other on a region basis, to select or extract a region having a smaller noise and achieve a composition based on the selected or extracted region. Alternatively, the image composition unit 233c may perform a composition by comparing the corrected images A and B with each other on a region basis and applying a greater weight to a region having a smaller noise to obtain the weighed sum. In this regard, the comparison between regions of the corrected images A and B may be performed on a per pixel basis or on the basis of a unit greater than the pixel, or regions divided according to the characteristics of an object may be compared. Hereinafter, a case in which regions divided according to the characteristics of an object are compared will be described.

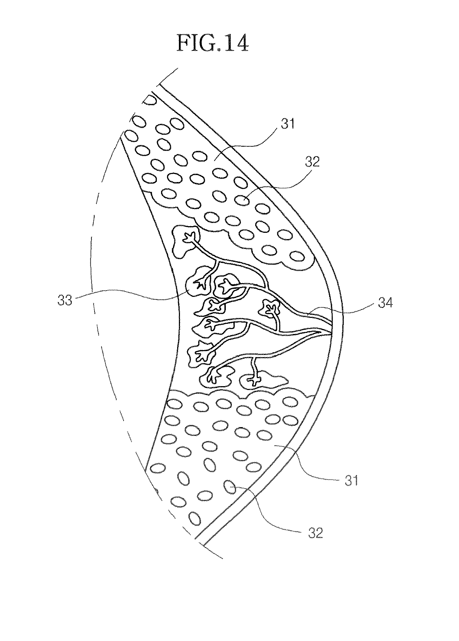

FIG. 14 is a sectional view illustrating a structure of inner tissues of a breast.

In an exemplary embodiment, a breast 30 as an object may be radiographed. Referring to FIG. 14, tissues of the breast 30 consist of a fibrous tissue 31 to enclose the breast 30 and maintain the shape of the breast 30, fatty tissues 32 distributed in the entire region of the breast 30, glandular tissues 33 to produce breast milk, and ductile tissues 34 through which breast milk is transported. Among these tissues, the glandular tissues 33 and the ductile tissues 34 involved in production and supply of breast milk are referred to as breast parenchyma. The parenchyma and the fatty tissues 32 exhibit different X-ray attenuation characteristics and thus characteristics of their X-ray images are also different.

Thus, the image composition unit 233c may divide an X-ray image of an object into a parenchyma region image and a fatty tissue region image, compare whether noise of the parenchyma region image is less in corrected image A or corrected image B to extract the parenchyma region image from one of the corrected images A and B which has less noise, compare whether noise of the fatty tissue region image is less in the corrected image A or the corrected image B to extract the fatty tissue region image from one of the corrected images A and B which has less noise, and compose the two region images.

Alternatively, a weight may be applied for each region such that a greater weight is applied to a corrected image having relatively less noise.

The corrected image obtained by composition is subjected to image processing by the image processor 234 and then is displayed on the display unit 241.

Hereinafter, an exemplary embodiment of a method for controlling the X-ray imaging apparatus described above will be described.

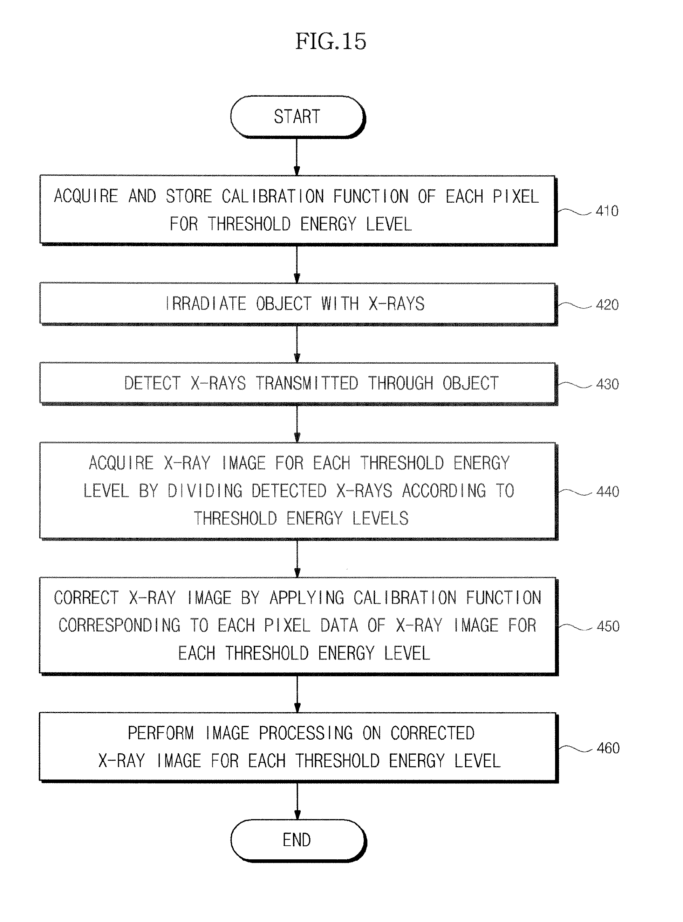

FIG. 15 is a flowchart for explaining a method of controlling an X-ray imaging apparatus, according to an exemplary embodiment.

Referring to FIG. 15, first, calibration functions of respective pixels for each threshold energy are acquired and stored (operation 410). Acquisition and storage of the calibration functions may be performed before radiography of an actual object, and may be performed once or periodically before use of the X-ray imaging apparatus, or may be performed whenever components of the X-ray imaging apparatus are replaced or without a predetermined cycle. In the present embodiment, the acquisition time or number of the calibration functions is not limited.

Subsequently, X-rays are irradiated to an actual object (operation 420), and X-rays transmitted through the object are detected (operation 430). In an embodiment, to acquire a multiple energy X-ray image having a high contrast between tissues, broadband X-rays having a plurality of energy bands may be irradiated to the object and detected.

Detected X-rays are divided according to threshold energies to acquire X-ray images for each threshold energy level (operation 440). In particular, when two threshold energy levels are input to each pixel of an X-ray detector of the X-ray imaging apparatus, two X-ray images corresponding to the two threshold energy levels are acquired. When three threshold energy levels are input to each pixel of the X-ray detector, three X-ray images corresponding thereto are acquired.

The X-ray images are corrected by applying, to the each pixel data thereof, a calibration function corresponding to each pixel data of the X-ray images according to the threshold energy levels (operation 450). For this operation, the calibration functions of respective pixels for each threshold energy level stored in operation 410 are called and applied to the corresponding pixel data. The image correction using calibration functions are the same as described above for the X-ray imaging apparatus 100, and thus a detailed description thereof is omitted.

Image processing is performed on the corrected X-ray images for each threshold energy level (operation 460), and a final image may be displayed on a display unit. In this regard, the image processing includes at least one of image processing for image quality improvement or image enhancement and image processing for acquisition of a multiple-energy X-ray image. As for the latter image processing, X-rays detected by the X-ray detector are divided according to a plurality of energy bands. This process has already been described above for the X-ray imaging apparatus 100 and thus a detailed description thereof is omitted.

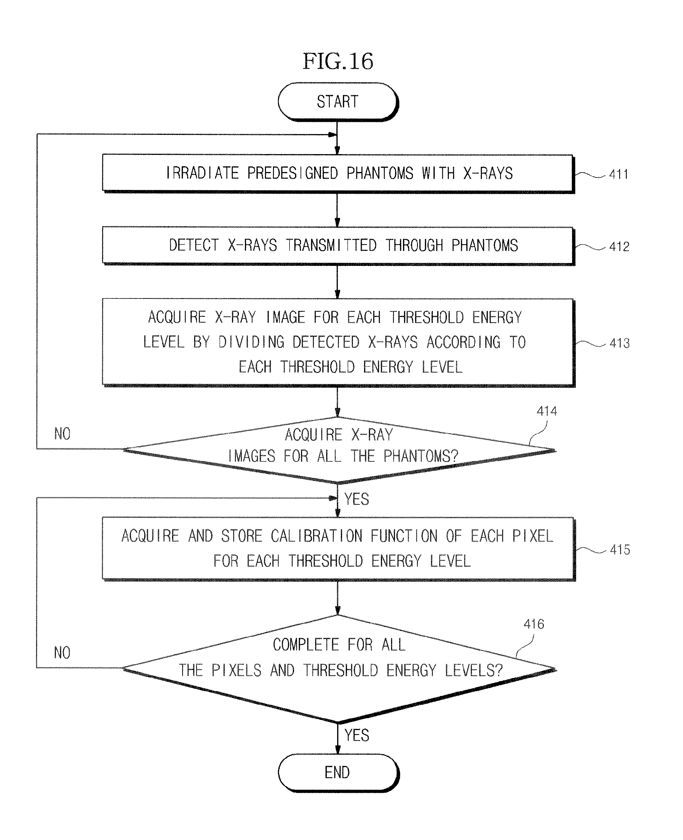

FIG. 16 is a flowchart specifically illustrating a process of acquiring and storing calibration functions of respective pixels for each threshold energy level.

Referring to FIG. 16, first, X-rays are irradiated to predesigned phantoms (operation 411) and X-rays transmitted through the phantoms are detected (operation 412). The phantoms may cover all the pixels of the X-ray detector 120 and be designed to have different thicknesses and material compositions, each of which has uniform thickness and material composition with respect to all the pixel regions.

The X-rays may be irradiated to the phantoms under conditions applied to radiography of an actual object. In this regard, the radiography may be performed for sufficient X-ray exposure time to minimize impact of quantum noise. The radiography conditions of an actual object include at least one of a tube voltage and tube current of an X-ray generator, a target material of a positive electrode, X-ray exposure time, the type of a filter, and threshold energy input to the X-ray detector.

Detected X-rays are divided according to threshold energies to acquire X-ray images for each threshold energy level (operation 413). The acquired X-ray images are stored in a measurement value storage unit.

When X-ray images for all the phantoms are acquired (operation 414), calibration functions of respective pixels for each threshold energy level are acquired using the stored X-ray images of the phantoms and stored (operation 415). The x-ray images consist of X-ray data corresponding to each pixel, and thus, the calibration functions of respective pixels for each threshold energy level may be acquired using the stored X-ray data. For example, assuming that a calibration function is a polynomial defined by a plurality of coefficients, unidentified coefficients may be determined by solving an equation by substituting the stored X-ray data into the polynomial, for each phantom. The acquisition and storage of the calibration functions have already been described in the above-described embodiment.

The acquisition and storage of the calibration functions are repeated until they are completed for all the pixels and all the threshold energy levels (operation 416). In the above-described exemplary embodiment, calibration functions are acquired for each threshold energy level. When radiography conditions are adjusted using an AEC of the X-ray imaging apparatus, however, calibration functions may be acquired for each condition by predicting several conditions that are applicable to an actual object. The radiography conditions may also include threshold energy.

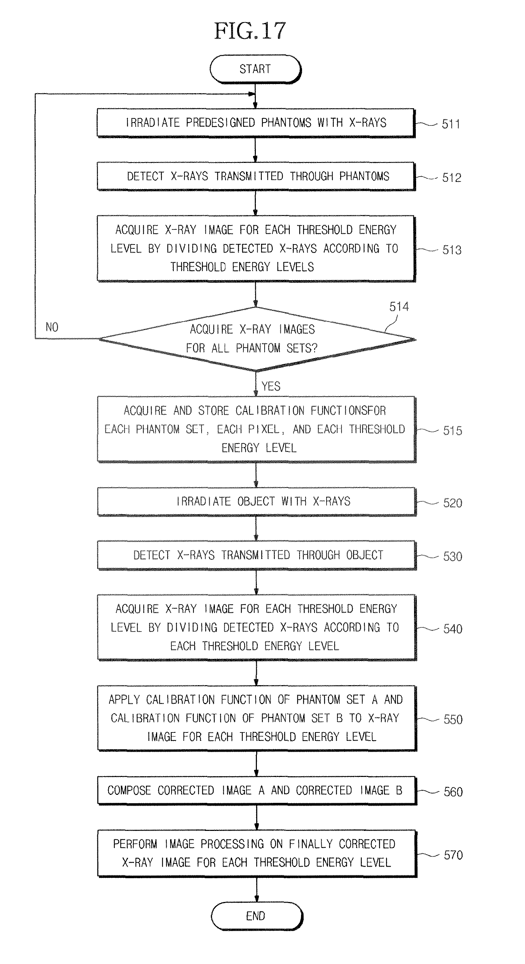

FIG. 17 is a flowchart for explaining a method of controlling an X-ray imaging apparatus, according to another exemplary embodiment. In the present exemplary embodiment, at least two types of calibration functions are acquired according to phantom sets.

Referring to FIG. 17, predesigned phantoms are irradiated with X-rays (operation 511), and X-rays transmitted through the phantoms are detected (operation 512). The X-rays may be irradiated to the phantoms under conditions applied to radiography of an actual object. The phantoms may cover all the pixels of the X-ray detector 120 and be designed to have different thicknesses and material compositions, each of which has uniform thickness and material composition with respect to all the pixel regions.

Detected X-rays are divided according to threshold energies to acquire X-ray images for threshold energy levels (operation 513) and the acquired X-ray images are stored.

When acquisition of X-ray images is completed for all phantom sets (operation 514), calibration functions for each phantom set, each pixel, and each threshold energy level are acquired and stored (operation 515). A single phantom set consists of a plurality of phantoms used to acquire a single calibration function, and the number of the phantom sets is not limited so as long at least two phantom sets are configured. In the present embodiment, however, two phantom sets (i.e., phantom set A and phantom set B) are used.

The acquisition and storage processes of calibration functions may be performed in the same manner as described in the above-described exemplary embodiment, except that the processes are performed for two phantom sets to acquire two calibration functions of the corresponding pixels for each threshold energy level and store the calibration functions.

When the acquisition and storage processes are completed, an actual object is radiographed and an X-ray image thereof is corrected.

For this operation, the object is irradiated with X-rays (operation 520), and X-rays transmitted through the object are detected (operation 530). In an embodiment, to acquire a multiple energy X-ray image having a high contrast between tissues, broadband X-rays having a plurality of energy bands may be irradiated to the object and detected.

Detected X-rays are divided according to threshold energies to acquire X-ray images for each threshold energy level (operation 540).

A calibration function of the phantom set A and a calibration function of the phantom set B are applied to the X-ray images for each threshold energy level (operation 550) to form corrected image A and corrected image B for each threshold energy level.

The corrected images A and B are composed (operation 560) to form a single corrected image. In particular, images of regions of the corrected images A and B which have the least noise may be selected or extracted through comparison therebetween to compose the selected or extracted images, or a greater weight may be applied to regions having relatively less noise to compose the weighted regions. In this regard, the comparison between regions of the corrected images A and B may be performed on a per pixel basis or on the basis of a unit greater than the pixel, or regions divided according to the characteristics of the object may be compared.

Lastly, the finally corrected X-ray image for each threshold energy level is subjected to image processing (operation 570) and the final X-ray image is displayed on a display unit. The image processing operation has already been described in the above-described embodiments.

As is apparent from the above description, according to an X-ray imaging apparatus and a method for controlling the X-ray imaging apparatus, mapping functions for correcting errors are calculated for each pixel and stored, and errors according to characteristics of each pixel may be corrected by applying the mapping functions to an output value of each pixel.

The disclosure discloses the following further aspects:

The first aspect of an X-ray imaging apparatus comprising: an X-ray generator configured to generate X-rays and irradiate the generated X-rays toward an object; an X-ray detector configured to detect the irradiated X-rays and output X-ray data by counting a number of photons having an energy level that is equal to or greater than a threshold energy level, among photons contained in the detected X-rays, for each of a plurality of pixels of the X-ray detector; a function acquisition unit configured to determine calibration functions for the plurality of pixels using X-ray data obtained from a plurality of predesigned phantoms; and an image correction unit configured to correct an X-ray image of the object on a per pixel basis using the determined calibration functions.

The X-ray imaging apparatus according to the first aspect, wherein the function acquisition unit comprises: a measurement value storage unit configured to store X-ray data output by the X-ray detector after the X-ray generator irradiates the plurality of predesigned phantoms with X-rays and the X-ray detector detects X-rays transmitted through the plurality of predesigned phantoms; and a calculator configured to perform calculation to determine the calibration functions using the X-ray data stored in the measurement value storage unit.

The X-ray imaging apparatus according to the second aspect, wherein one of the acquired calibration functions is a function defined by at least one coefficient and the calculator substitutes the X-ray data stored in the measurement value storage unit for a variable of the function to determine a value of the at least one coefficient.

The X-ray imaging apparatus according to the third aspect, wherein the calculator determines the value of the at least one coefficient by assuming that ideal X-ray data with no errors are a value of the function.

The X-ray imaging apparatus according to the fourth aspect, wherein the calculator determines the value of the at least one coefficient by substituting a representative value of the X-ray data for the plurality of predesigned phantoms for the value of the function.

The X-ray imaging apparatus according to the fifth aspect, wherein the representative value of the X-ray data for the plurality of predesigned phantoms comprises at least one selected from a group consisting of the most frequent value among all pixel values, a median of the all pixel values, and an average of a weighted sum obtained by applying a corresponding weight to each of all pixel values and the all pixel values.

The X-ray imaging apparatus according to the first aspect, wherein, when at least two threshold energy levels are input to a single pixel of the X-ray detector, the function acquisition unit acquires the calibration function for each of the at least two threshold energy levels.

The X-ray imaging apparatus according to the seventh aspect, wherein the image correction unit corrects the X-ray image of the object on a per pixel basis for each of the two threshold energy levels using the calibration function when the at least two threshold energy levels are input to the single pixel of the X-ray detector.

The X-ray imaging apparatus according to the first aspect, wherein the function acquisition unit acquires the calibration functions for each of a plurality of radiography conditions of the object.

The X-ray imaging apparatus according to the ninth aspect, wherein the image correction unit corrects the X-ray image of the object using the calibration functions corresponding to each of a plurality of radiography conditions of the object.

The X-ray imaging apparatus according the first aspect, wherein the function acquisition unit divides the plurality of predesigned phantoms into at least two phantom sets and acquires the calibration functions for the at least two phantom sets.

The X-ray imaging apparatus according to the eleventh aspect, wherein the image correction unit forms at least two corrected X-ray images by applying the calibration functions acquired for the at least two phantom sets to the X-ray image of the object.

The X-ray imaging apparatus according to the twelfth aspect, wherein the image correction unit forms a single corrected X-ray image based on the at least two corrected X-ray images.

The X-ray imaging apparatus according to the thirteenth aspect, wherein the image correction unit selects regions having least noise through comparison between the at least two corrected X-ray images on a region basis and composes the regions, or

applies a greater weight to regions having less noise and composes the weighted regions.

The X-ray imaging apparatus according to the fourteenth aspect, wherein the regions are one of regions on a pixel basis and regions divided according to characteristics of the object.

The aspect of a method of controlling an X-ray imaging apparatus, the method comprising: determining calibration functions for a plurality of pixels using X-ray data obtained from a plurality of predesigned phantoms; irradiating an object with X-rays and detecting X-rays transmitted through the object to acquire an X-ray image of the object; and correcting the X-ray image of the object on a per pixel basis using the determined calibration functions.

The method according to the sixteenth aspect, wherein the determining comprises:

storing the X-ray data obtained from the plurality of predesigned phantoms; and
performing calculation to determine the calibration functions using the stored X-ray data.

The method according to the seventeenth aspect, wherein one of the acquired calibration functions is a function defined by at least one coefficient and the performing calculation substitutes the stored X-ray data for a variable of the function to determine a value of the at least one coefficient.

The method according to the eighteenth aspect, wherein the performing comprises determining the value of the at least one coefficient by assuming that ideal X-ray data with no errors are a value of the function.

The method according to the nineteenth aspect, wherein the performing comprises determining the value of the at least one coefficient by substituting a representative value of the X-ray data for the plurality of predesigned phantoms for the value of the function.

The method according to the twentieth aspect, wherein the representative value of the X-ray data for the plurality of predesigned phantoms comprises at least one selected from a group consisting of the most frequent value among all pixel values, a median of the all pixel values, and an average of a weighted sum obtained by applying a corresponding weight to each pixel value and the all pixel values.

The method according to the sixteenth aspect, wherein the acquiring comprises, when the X-ray image of the object is acquired for each of at least two threshold energy levels, acquiring the calibration function for each of the at least two threshold energy levels.

The method according to the twenty-second aspect, wherein the correcting comprises, when the X-ray image of the object is acquired for each of the at least two threshold energy levels, correcting the X-ray image of the object on a per pixel basis for each of the at least two threshold energy levels using the calibration functions.

The method according to the sixteenth aspect, wherein the acquiring comprises acquiring the calibration functions for each of a plurality of radiography conditions of the object.

The method according to the twenty-fourth aspect, wherein the correcting comprises correcting the X-ray image of the object using calibration functions corresponding to each of a plurality of radiography conditions applied to the object among the acquired calibration functions.

The method according to the sixteenth aspect, wherein the acquiring comprises dividing the plurality of predesigned phantoms into at least two phantom sets and acquiring the calibration functions for the at least two phantom sets.

The method according to the twenty-sixth aspect, wherein the correcting comprises forming at least two corrected X-ray images by applying the calibration functions acquired for the at least two phantom sets to the X-ray image of the object.

The method according to the twenty-seventh aspect, wherein the correcting further comprises forming a single corrected X-ray image based on the at least two corrected X-ray images.

The method according to the twenty-eighth aspect, wherein the composing comprises selecting regions having least noise through comparison between the at least two corrected X-ray images on a region basis and composing the regions, or applying a greater weight to regions having less noise and composing the weighted regions.

The method according to the twenty-ninth aspect, wherein the regions are one of regions on a pixel basis and regions divided according to characteristics of the object. The aspect of a method of processing an X-ray image, the method comprising:

calibrating an x-ray detector, the calibrating comprising calculating calibration functions for a plurality of image areas by correlating measured X-ray data obtained from a plurality of predetermined imaging phantoms to estimated x-ray data for the plurality of predetermined imaging phantoms; emitting X-rays toward an object and detecting X-rays transmitted through the object to capture an X-ray image of the object; and adjusting the X-ray image of the object in units of image areas based on the calculated calibration functions.

The method of the thirty-first aspect, wherein the calibration functions are obtained for each of at least two sets of imaging phantoms of the plurality of predetermined imaging phantom, for each of the plurality of image areas, and for each of a plurality of threshold energy levels.

**Claims**

1.  An X-ray imaging apparatus comprising:

    an X-ray generator configured to generate X-rays and irradiate the generated X-rays;
    an X-ray detector configured to detect the irradiated X-rays and output X-ray data by counting a number of photons having an energy level that is equal to or greater than a threshold energy level, among photons contained in the detected X-rays, for each of a plurality of pixels of the X-ray detector;
    a function acquisition unit configured to determine calibration functions for the plurality of pixels using X-ray data obtained from a plurality of predesigned phantoms; and
    an image correction unit configured to correct an X-ray image of the object on a per pixel basis using the determined calibration functions.

2.  The X-ray imaging apparatus according to claim 1, wherein the function acquisition unit comprises:

    a measurement value storage unit configured to store X-ray data output by the X-ray detector after the X-ray generator irradiates the plurality of predesigned phantoms with X-rays and the X-ray detector detects X-rays transmitted through the plurality of predesigned phantoms; and
    a calculator configured to perform calculation to determine the calibration functions using the X-ray data stored in the measurement value storage unit.

3.  The X-ray imaging apparatus according to claim 2, wherein one of the acquired calibration functions is a function defined by at least one coefficient and the calculator substitutes the X-ray data stored in the measurement value storage unit for a variable of the function to determine a value of the at least one coefficient,
    wherein preferably the calculator determines the value of the at least one coefficient by assuming that ideal X-ray data with no errors are a value of the function.

4.  The X-ray imaging apparatus according to claim 3, wherein the calculator determines the value of the at least one coefficient by substituting a representative value of the X-ray data for the plurality of predesigned phantoms for the value of the function,
    wherein preferably the representative value of the X-ray data for the plurality of predesigned phantoms comprises at least one selected from a group consisting of the most frequent value among all pixel values, a median of the all pixel values, and an average of a weighted sum obtained by applying a corresponding weight to each of all pixel values and the all pixel values.

5.  The X-ray imaging apparatus according to claim 1, wherein, when at least two threshold energy levels are input to a single pixel of the X-ray detector, the function acquisition unit acquires the calibration function for each of the at least two threshold energy levels,
    wherein preferably the image correction unit corrects the X-ray image of the object on a per pixel basis for each of the two threshold energy levels using the calibration function when the at least two threshold energy levels are input to the single pixel of the X-ray detector.

6.  The X-ray imaging apparatus according to claim 1, wherein the function acquisition unit acquires the calibration functions for each of a plurality of radiography conditions of the object,
    wherein preferably the image correction unit corrects the X-ray image of the object using the calibration functions corresponding to each of a plurality of radiography conditions of the object.

**7.** The X-ray imaging apparatus according to claim 1, wherein the function acquisition unit divides the plurality of predesigned phantoms into at least two phantom sets and acquires the calibration functions for the at least two phantom sets,

wherein preferably the image correction unit forms at least two corrected X-ray images by applying the calibration functions acquired for the at least two phantom sets to the X-ray image of the object.

**8.** The X-ray imaging apparatus according to claim 7, wherein the image correction unit forms a single corrected X-ray image based on the at least two corrected X-ray images,

wherein preferably the image correction unit selects regions having least noise through comparison between the at least two corrected X-ray images on a region basis and preferably composes the regions, or applies a greater weight to regions having less noise and composes the weighted regions,

wherein preferably the regions are one of regions on a pixel basis and regions divided according to characteristics of the object.

**9.** A method of controlling an X-ray imaging apparatus, the method comprising:

determining calibration functions for a plurality of pixels using X-ray data obtained from a plurality of predesigned phantoms;

irradiating an object with X-rays and detecting X-rays transmitted through the object to acquire an X-ray image of the object; and

correcting the X-ray image of the object on a per pixel basis using the determined calibration functions.

**10.** The method according to claim 10, wherein the determining comprises:

storing the X-ray data obtained from the plurality of predesigned phantoms; and

performing calculation to determine the calibration functions using the stored X-ray data.

**11.** The method according to claim 10, wherein one of the acquired calibration functions is a function defined by at least one coefficient and the performing calculation substitutes the stored X-ray data for a variable of the function to determine a value of the at least one coefficient,

wherein preferably the performing comprises determining the value of the at least one coefficient by assuming that ideal X-ray data with no errors are a value of the function.

**12.** The method according to claim 11, wherein the performing comprises determining the value of the at least one coefficient by substituting a representative value of the X-ray data for the plurality of predesigned phantoms for the value of the function,

wherein preferably the representative value of the X-ray data for the plurality of predesigned phantoms comprises at least one selected from a group consisting of the most frequent value among all pixel values, a median of the all pixel values, and an average of a weighted sum obtained by applying a corresponding weight to each pixel value and the all pixel values.

**13.** The method according to claim 9, wherein the acquiring comprises, when the X-ray image of the object is acquired for each of at least two threshold energy levels, acquiring the calibration function for each of the at least two threshold energy levels,

wherein preferably the correcting comprises, when the X-ray image of the object is acquired for each of the at least two threshold energy levels, correcting the X-ray image of the object on a per pixel basis for each of the at least two threshold energy levels using the calibration functions.

**14.** The method according to claim 9, wherein the acquiring comprises acquiring the calibration functions for each of a plurality of radiography conditions of the object,

wherein preferably the correcting comprises correcting the X-ray image of the object using calibration functions corresponding to each of a plurality of radiography conditions applied to the object among the acquired calibration functions.

**15.** The method according to claim 9, wherein the acquiring comprises dividing the plurality of predesigned phantoms into at least two phantom sets and acquiring the calibration functions for the at least two phantom sets,

wherein preferably the correcting comprises forming at least two corrected X-ray images by applying the calibration functions acquired for the at least two phantom sets to the X-ray image of the object,

wherein preferably the correcting further comprises forming a single corrected X-ray image based on the at least two corrected X-ray images,
wherein preferably the composing comprises selecting regions having least noise through comparison between the at least two corrected X-ray images on a region basis and composing the regions, or applying a greater weight to regions having less noise and composing the weighted regions,
wherein preferably the regions are one of regions on a pixel basis and regions divided according to characteristics of the object.

FIG. 1

FIG.2

FIG.3

FIG.4A

LIGHT RECEIVING ELEMENT(121)                    READOUT CIRCUIT(122)

X-ray

PHOTON

PREAMPLIFIER

COMPARATOR

COUNTER

122c

+

−

THRESHOLD VOLTAGE(V th)

123

122a

122b

FIG.4B

LIGHT RECEIVING ELEMENT(121)

READOUT CIRCUIT(122)

X-ray

PHOTON

123

PREAMPLIFIER

122a

COMPARATOR 1

122b-1

THRESHOLD VOLTAGE ($V_{th1}$)

122c-1

COUNTER 1

COMPARATOR 2

122b-2

THRESHOLD VOLTAGE ($V_{th2}$)

122c-2

COUNTER 2

COMPARATOR 3

122b-3

THRESHOLD VOLTAGE 1($V_{th3}$)

122c-3

COUNTER 3

FIG.5A

# FIG.5B

FIG.6

FIG.7

100    130

110    131    132    141

X-RAY GENERATOR ← FUNCTION ACQUISITION UNIT → FUNCTION STORAGE UNIT    DISPLAY UNIT

X-RAY DETECTOR → IMAGE CORRECTION UNIT → IMAGE PROCESSOR

120    133    134

27

FIG.8A

# FIG.8B

131

131a

MEASUREMENT VALUE STORAGE UNIT

CALCULATOR

131b

| | Phantom | | | | | | | |
|---|---|---|---|---|---|---|---|---|
| Th1 | Phantom 1 | $b^1_{1,11}$ | $b^1_{1,12}$ | $b^1_{1,13}$ | $\cdots$ | $b^1_{1,21}$ | $b^1_{1,22}$ | $\cdots$ | $b^1_{mn}$ |
| | Phantom 2 | $b^2_{1,11}$ | $b^2_{1,12}$ | $b^2_{1,13}$ | $\cdots$ | $b^2_{1,21}$ | $b^2_{1,22}$ | $\cdots$ | $b^2_{mn}$ |
| | $\vdots$ | | | | | | | |
| | Phantom k | $b^k_{1,11}$ | $b^k_{1,12}$ | $b^k_{1,13}$ | $\cdots$ | $b^k_{1,21}$ | $b^k_{1,22}$ | $\cdots$ | $b^k_{mn}$ |
| Th2 | Phantom 1 | $b^1_{2,11}$ | $b^1_{2,12}$ | $b^1_{2,13}$ | $\cdots$ | $b^1_{2,21}$ | $b^1_{2,22}$ | $\cdots$ | $b^1_{mn}$ |
| | Phantom 2 | $b^2_{2,11}$ | $b^2_{2,12}$ | $b^2_{2,13}$ | $\cdots$ | $b^2_{2,21}$ | $b^2_{2,22}$ | $\cdots$ | $b^2_{mn}$ |
| | $\vdots$ | | | | | | | |
| | Phantom k | $b^k_{2,11}$ | $b^k_{2,12}$ | $b^k_{2,13}$ | $\cdots$ | $b^k_{2,21}$ | $b^k_{2,22}$ | $\cdots$ | $b^k_{mn}$ |
| Th3 | Phantom 1 | $b^1_{3,11}$ | $b^1_{3,12}$ | $b^1_{3,13}$ | $\cdots$ | $b^1_{3,21}$ | $b^1_{3,22}$ | $\cdots$ | $b^1_{mn}$ |
| | Phantom 2 | $b^2_{3,11}$ | $b^2_{3,12}$ | $b^2_{3,13}$ | $\cdots$ | $b^2_{3,21}$ | $b^2_{3,22}$ | $\cdots$ | $b^2_{mn}$ |
| | $\vdots$ | | | | | | | |
| | Phantom k | $b^k_{3,11}$ | $b^k_{3,12}$ | $b^k_{3,13}$ | $\cdots$ | $b^k_{3,21}$ | $b^k_{3,22}$ | $\cdots$ | $b^k_{mn}$ |

# FIG.9

| | PX$_{11}$ | f$_{1,11}$(b) |
|---|---|---|
| | PX$_{12}$ | f$_{1,12}$(b) |
| | ⋮ | ⋮ |
| Th1 | PX$_{21}$ | f$_{1,21}$(b) |
| | ⋮ | ⋮ |
| | PX$_{mn}$ | f$_{1,mn}$(b) |
| | PX$_{11}$ | f$_{2,11}$(b) |
| | PX$_{12}$ | f$_{2,12}$(b) |
| | ⋮ | ⋮ |
| Th2 | PX$_{21}$ | f$_{2,21}$(b) |
| | ⋮ | ⋮ |
| | PX$_{mn}$ | f$_{2,mn}$(b) |
| | PX$_{11}$ | f$_{3,11}$(b) |
| | PX$_{12}$ | f$_{3,12}$(b) |
| | ⋮ | ⋮ |
| Th3 | PX$_{21}$ | f$_{3,21}$(b) |
| | ⋮ | ⋮ |
| | PX$_{mn}$ | f$_{3,mn}$(b) |

132

FUNCTION STORAGE UNIT

FIG.10

| | | $b^1_{1,11}$ | $b^1_{1,12}$ | $b^1_{1,13}$ | $\cdots$ | $b^1_{1,21}$ | $b^1_{1,22}$ | $\cdots$ | $b^1_{mn}$ |
|---|---|---|---|---|---|---|---|---|---|
| | Phantom 1 | $b^1_{1,11}$ | $b^1_{1,12}$ | $b^1_{1,13}$ | $\cdots$ | $b^1_{1,21}$ | $b^1_{1,22}$ | $\cdots$ | $b^1_{mn}$ |
| CONDITION 1 | Phantom 2 | $b^2_{1,11}$ | $b^2_{1,12}$ | $b^2_{1,13}$ | $\cdots$ | $b^2_{1,21}$ | $b^2_{1,22}$ | $\cdots$ | $b^2_{mn}$ |
| | $\vdots$ | | | | | | | | |
| | Phantom k | $b^k_{1,11}$ | $b^k_{1,12}$ | $b^k_{1,13}$ | $\cdots$ | $b^k_{1,21}$ | $b^k_{1,22}$ | $\cdots$ | $b^k_{mn}$ |
| | Phantom 1 | $b^1_{2,11}$ | $b^1_{2,12}$ | $b^1_{2,13}$ | $\cdots$ | $b^1_{2,21}$ | $b^1_{2,22}$ | $\cdots$ | $b^1_{mn}$ |
| CONDITION 2 | Phantom 2 | $b^2_{2,11}$ | $b^2_{2,12}$ | $b^2_{2,13}$ | $\cdots$ | $b^2_{2,21}$ | $b^2_{2,22}$ | $\cdots$ | $b^2_{mn}$ |
| | $\vdots$ | | | | | | | | |
| | Phantom k | $b^k_{2,11}$ | $b^k_{2,12}$ | $b^k_{2,13}$ | $\cdots$ | $b^k_{2,21}$ | $b^k_{2,22}$ | $\cdots$ | $b^k_{mn}$ |
| | Phantom 1 | $b^1_{3,11}$ | $b^1_{3,12}$ | $b^1_{3,13}$ | $\cdots$ | $b^1_{3,21}$ | $b^1_{3,22}$ | $\cdots$ | $b^1_{mn}$ |
| CONDITION 3 | Phantom 2 | $b^2_{3,11}$ | $b^2_{3,12}$ | $b^2_{3,13}$ | $\cdots$ | $b^2_{3,21}$ | $b^2_{3,22}$ | $\cdots$ | $b^2_{mn}$ |
| | $\vdots$ | | | | | | | | |
| | Phantom k | $b^k_{3,11}$ | $b^k_{3,12}$ | $b^k_{3,13}$ | $\cdots$ | $b^k_{3,21}$ | $b^k_{3,22}$ | $\cdots$ | $b^k_{mn}$ |

131

131a

MEASUREMENT VALUE STORAGE UNIT

CALCULATOR

131b

EP 2 664 280 A2

FIG.11

| | | PX$_{11}$ | f$_{1,11}$(b) |
|---|---|---|---|
| | | PX$_{12}$ | f$_{1,12}$(b) |
| CONDITION 1 | | ⋮ | ⋮ |
| | | PX$_{21}$ | f$_{1,21}$(b) |
| | | ⋮ | ⋮ |
| | | PX$_{mn}$ | f$_{1,mn}$(b) |
| | | PX$_{11}$ | f$_{2,11}$(b) |
| | | PX$_{12}$ | f$_{2,12}$(b) |
| CONDITION 2 | | ⋮ | ⋮ |
| | | PX$_{21}$ | f$_{2,21}$(b) |
| | | ⋮ | ⋮ |
| | | PX$_{mn}$ | f$_{2,mn}$(b) |
| | | PX$_{11}$ | f$_{3,11}$(b) |
| | | PX$_{12}$ | f$_{3,12}$(b) |
| CONDITION 3 | | ⋮ | ⋮ |
| | | PX$_{21}$ | f$_{3,21}$(b) |
| | | ⋮ | ⋮ |
| | | PX$_{mn}$ | f$_{3,mn}$(b) |

132

FUNCTION STORAGE UNIT

FIG.12

EP 2 664 280 A2

EP 2 664 280 A2

## FIG.13

| | | |
|---|---|---|
| **Th1** | $PX_{11}$ | $f^A_{1,11}(b)$, $f^B_{1,11}(b)$ |
| | $PX_{12}$ | $f^A_{1,12}(b)$, $f^B_{1,12}(b)$ |
| | $\vdots$ | $\vdots$ |
| | $PX_{21}$ | $f^A_{1,21}(b)$, $f^B_{1,21}(b)$ |
| | $\vdots$ | $\vdots$ |
| | $PX_{mn}$ | $f^A_{1,mn}(b)$, $f^B_{1,mn}(b)$ |
| **Th2** | $PX_{11}$ | $f^A_{2,11}(b)$, $f^B_{2,11}(b)$ |
| | $PX_{12}$ | $f^A_{2,12}(b)$, $f^B_{2,12}(b)$ |
| | $\vdots$ | $\vdots$ |
| | $PX_{21}$ | $f^A_{2,21}(b)$, $f^B_{2,21}(b)$ |
| | $\vdots$ | $\vdots$ |
| | $PX_{mn}$ | $f^A_{2,mn}(b)$, $f^B_{2,mn}(b)$ |
| **Th3** | $PX_{11}$ | $f^A_{3,11}(b)$, $f^B_{3,11}(b)$ |
| | $PX_{12}$ | $f^A_{3,12}(b)$, $f^B_{3,12}(b)$ |
| | $\vdots$ | $\vdots$ |
| | $PX_{21}$ | $f^A_{3,21}(b)$, $f^B_{3,21}(b)$ |
| | $\vdots$ | $\vdots$ |
| | $PX_{mn}$ | $f^A_{3,mn}(b)$, $f^B_{3,mn}(b)$ |

232

FUNCTION
STORAGE UNIT

FIG.14

## FIG.15

START

ACQUIRE AND STORE CALIBRATION FUNCTION OF EACH PIXEL
FOR THRESHOLD ENERGY LEVEL — 410

IRRADIATE OBJECT WITH X-RAYS — 420

DETECT X-RAYS TRANSMITTED THROUGH OBJECT — 430

ACQUIRE X-RAY IMAGE FOR EACH THRESHOLD ENERGY
LEVEL BY DIVIDING DETECTED X-RAYS ACCORDING TO
THRESHOLD ENERGY LEVELS — 440

CORRECT X-RAY IMAGE BY APPLYING CALIBRATION FUNCTION
CORRESPONDING TO EACH PIXEL DATA OF X-RAY IMAGE FOR
EACH THRESHOLD ENERGY LEVEL — 450

PERFORM IMAGE PROCESSING ON CORRECTED
X-RAY IMAGE FOR EACH THRESHOLD ENERGY LEVEL — 460

END

## FIG.16

START

IRRADIATE PREDESIGNED PHANTOMS WITH X-RAYS ～411

DETECT X-RAYS TRANSMITTED THROUGH PHANTOMS ～412

ACQUIRE X-RAY IMAGE FOR EACH THRESHOLD ENERGY
LEVEL BY DIVIDING DETECTED X-RAYS ACCORDING TO
EACH THRESHOLD ENERGY LEVEL ～413

ACQUIRE X-RAY
IMAGES FOR ALL THE PHANTOMS? 414

NO

YES

ACQUIRE AND STORE CALIBRATION FUNCTION OF EACH PIXEL
FOR EACH THRESHOLD ENERGY LEVEL ～415

COMPLETE FOR ALL
THE PIXELS AND THRESHOLD ENERGY LEVELS? 416

NO

YES

END

# FIG.17

START

IRRADIATE PREDESIGNED PHANTOMS WITH X-RAYS — 511

DETECT X-RAYS TRANSMITTED THROUGH PHANTOMS — 512

ACQUIRE X-RAY IMAGE FOR EACH THRESHOLD ENERGY LEVEL BY DIVIDING DETECTED X-RAYS ACCORDING TO THRESHOLD ENERGY LEVELS — 513

514

ACQUIRE X-RAY IMAGES FOR ALL PHANTOM SETS?

NO

YES

ACQUIRE AND STORE CALIBRATION FUNCTIONSFOR EACH PHANTOM SET, EACH PIXEL, AND EACH THRESHOLD ENERGY LEVEL — 515

IRRADIATE OBJECT WITH X-RAYS — 520

DETECT X-RAYS TRANSMITTED THROUGH OBJECT — 530

ACQUIRE X-RAY IMAGE FOR EACH THRESHOLD ENERGY LEVEL BY DIVIDING DETECTED X-RAYS ACCORDING TO EACH THRESHOLD ENERGY LEVEL — 540

APPLY CALIBRATION FUNCTION OF PHANTOM SET A AND CALIBRATION FUNCTION OF PHANTOM SET B TO X-RAY IMAGE FOR EACH THRESHOLD ENERGY LEVEL — 550

COMPOSE CORRECTED IMAGE A AND CORRECTED IMAGE B — 560

PERFORM IMAGE PROCESSING ON FINALLY CORRECTED X-RAY IMAGE FOR EACH THRESHOLD ENERGY LEVEL — 570

END